# EUROPEAN PATENT SPECIFICATION

(11) **EP 3 260 157 B1**
(45) Date of publication and mention of the grant of the patent: **03.04.2024**
(21) Application number: 16752524.5
(22) Date of filing: 17.02.2016
(51) Int. Cl.: A61M 39/06, A61M 25/06, A61M 5/158, A61M 5/50

(54) **INDWELLING NEEDLE ASSEMBLY**
DAUERNADELANORDNUNG
ENSEMBLE AIGUILLE À DEMEURE

(30) Priority: 17.02.2015 JP 2015028292
(43) Date of publication of application: 27.12.2017
(73) Proprietor: Nipro Corporation, Osaka-shi, Osaka 531-8510 (JP)
(72) Inventor: NAKAGAMI, Hiroyuki, Osaka-shi Osaka 531-8510 (JP); SAKAMOTO, Shingo, Osaka-shi Osaka 531-8510 (JP); MINO, Asumi, Osaka-shi Osaka 531-8510 (JP)
(74) Representative: Kraus & Lederer PartGmbB
(86) International application number: PCT/JP2016/054617
(87) International publication number: WO 2016/133138

(56) References cited:
- WO-A1-2007/049563
- WO-A1-2010/038471
- JP-A- 2013 524 884
- JP-A- 2014 517 729
- US-A1- 2013 030 391
- US-A1- 2014 025 009

## Description

### TECHNICAL FIELD

The present invention relates to an indwelling needle assembly used to puncture a blood vessel and to be left therein during fluid transfusion, blood collection, blood dialysis or the like. More particularly, the present invention pertains to an indwelling needle assembly including a needle tip protector for protecting a needle tip of an inner needle after use.

### BACKGROUND ART

From the past, there has been known an indwelling needle assembly wherein an inner needle unit and an outer needle unit are mutually assembled in a state an inner needle of the inner needle unit is inserted through an outer needle of the outer needle unit. When performing fluid transfusion, blood collection, or blood dialysis, for example, a blood vessel of the patient is punctured with the indwelling needle assembly. After the puncture, by removing the inner needle unit including the inner needle from the outer needle unit including the outer needle, the outer needle is detained in the blood vessel of the patient, in order to use it for the treatment.

It is preferable that the inner needle unit of this indwelling needle assembly includes a needle tip protector for covering the needle tip of the inner needle when the inner needle is extracted, in order to avoid inadvertent pricking by the inner needle removed from the outer needle unit after the puncture. Specifically, for an indwelling needle assembly disclosed in Japanese Patent No. JP-B-4994775 (Patent Document 1), by extracting the inner needle, the needle tip of the inner needle is positioned in a protection sleeve provided for the needle tip protector, so that inadvertent pricking of the inner needle after use is prevented.

For the indwelling needle assembly disclosed in Patent Document 1 referred above, in the initial state where the inner needle is inserted through the outer needle, the needle tip protector is made non-detachable from the outer needle hub by an engagement claw provided at the needle tip protector being engaged in relation to the outer peripheral face of the outer needle hub. As the inner needle is extracted, the engagement claw is separated radially outward from the outer needle hub, releasing connection between the needle tip protector and the outer needle hub by engagement action of the engagement claw, so that the needle tip of the inner needle in the inner needle unit separated from the outer needle unit is protected by the needle tip protector.

Unfortunately, in the indwelling needle assembly disclosed in Patent Document 1 described above, when drawing the inner needle inserted through the outer needle out of it, it is required to move the engagement claw toward the outer peripheral side of the outer needle hub, in order to release the engagement between the engagement claw and the outer needle hub. Therefore, a space must be secured on the outer peripheral side of the outer needle hub for allowing the movement of the engagement claw. In addition to that, the size of the indwelling needle assembly including the engagement claw after the movement to the outer peripheral side of the outer needle hub cannot be avoided from becoming large. Consequently, medical service workers may feel inconvenience for the work when performing a treatment using the indwelling needle assembly in relation to the patients. A further indwelling needle assembly is disclosed in document US 2013/030391 A1.

### BACKGROUND ART DOCUMENT

### PATENT DOCUMENT

Patent Document 1: JP-B-4994775

### SUMMARY OF THE INVENTION

### PROBLEM THE INVENTION ATTEMPTS TO SOLVE

The present invention has been developed in view of the above-described matters as the background, and it is an object of the present invention to provide an indwelling needle assembly with a novel structure capable of keeping a protection mechanism for a needle tip of an inner needle drawn out of an outer needle, avoiding enlargement of the indwelling needle assembly to an outer peripheral side of an outer needle hub, and facilitating the work for the treatment.

### MEANS FOR SOLVING THE PROBLEM

A first aspect of the present invention provides an indwelling needle assembly comprising: an inner needle having an inner needle hub on a proximal end side thereof; an outer needle having an outer needle hub on a proximal end side thereof, the outer needle receiving the inner needle being inserted from a proximal end side of the outer needle hub; a needle tip protector attached to the inner needle, the needle tip protector configured to move to a distal end of the inner needle so as to cover a needle tip of the inner needle; and a connection mechanism connecting the proximal end side of the outer needle hub to a distal end side of the needle tip protector, the indwelling needle assembly being characterized in that: a connection member is provided extending out from the needle tip protector to an inside of the outer needle hub while being abutted against an inner face of the outer needle hub so as to restrict movement of the outer needle hub relative to the needle tip protector and to keep a connected state of the outer needle hub to the needle tip protector by the connection mechanism, and a disconnection mechanism is provided and configured to move the connection member to an inner peripheral side of the outer needle hub so as to release the connected state when the inner needle is extracted from the outer needle.

According to the indwelling needle assembly having the structure of the present aspect, since the needle tip protector is connected to the outer needle hub by the connection mechanism, by moving the inner needle relative to the outer needle to a side of extraction from the outer needle, the needle tip protector is moved to the needle tip of the inner needle, so that the needle tip of the inner needle is protected rapidly and stably. Also, when the inner needle is drawn out from the outer needle, connection between the outer needle hub and the needle tip protector is released by the disconnection mechanism, so that the inner needle can be removed from the outer needle while being protected by the needle tip protector, leaving the outer needle in the skin of the patient.

Here, when disconnecting the outer needle hub and the needle tip protector, the connection member connecting them is moved to the inner peripheral side of the outer needle hub, thus eliminating the need for a special space to be kept on the outer peripheral side of the outer needle hub, while avoiding the size of the outer needle hub from becoming larger after the connection member is moved. This can improve easiness for work or operation in the treatment.

It is preferable that the connection member is configured to be automatically moved to the inner peripheral side of the outer needle hub using a mechanism that interlocks with the inner needle being extracted from the outer needle, for the disconnection between the outer needle hub and the needle tip protector. Alternatively, it is possible to use a structure wherein the engagement of the connection member in the outer needle hub is released by moving the connection member by hand manipulation after the inner needle is drawn out of the outer needle.

A second aspect of the present invention provides the indwelling needle assembly according to the first aspect, further comprising an urging means provided to always exert an urging force of a direction to move the connection member to the inner peripheral side of the outer needle hub.

According to the indwelling needle assembly having the structure of the present aspect, the connection member is always urged to the inner peripheral side of the outer needle hub by the urging means, thereby allowing the connection member to move to the inner peripheral side automatically when the inner needle is extracted from the outer needle. As a result, by drawing the inner needle out of the outer needle, the connection between the outer needle hub and the needle tip protector is automatically released, so that removal of the inner needle from the outer needle can be realized even more easily.

As a member constituting the urging means, for example, a material that exerts an elastic force as an urging force such as a metal spring, a resin spring, and a rubber elastic body, a magnet that exerts a magnetic force as the urging force, and the like can be adopted preferably.

A third aspect of the present invention provides the indwelling needle assembly according to the first or second aspect, further comprising an outer peripheral engagement part extending out from the needle tip protector to an outside of the outer needle hub so that the outer peripheral engagement part is abutted against an outer face of the outer needle hub as a peripheral wall of the outer needle hub is clamped between the outer peripheral engagement part and the connection member.

According to the indwelling needle assembly having the structure of the present aspect, the needle tip protector and the outer needle hub are connected in contact as the peripheral wall of the outer needle hub is clamped between the outer peripheral engagement part and the connection member, so that, in the connection, e.g., before the use of the indwelling needle assembly, excessive play etc. is also restrained, and the connected state can be maintained more stably. Since the connection member is moved to the inner peripheral side when the connected state is released, it is possible to certainly release clamping of the outer needle hub made by the connection member and the outer peripheral engagement part.

It is preferable to provide catch parts that are locked using concave and convex shapes to avoid axial detachment of the outer needle hub and the needle tip protector, at the contact section between the outer peripheral face of the outer needle hub and the outer peripheral engagement part and/or the contact section between the inner peripheral face of the outer needle hub and the connection member. This makes it possible to more stably keep the connected state between the outer needle hub and the needle tip protector.

It is also preferable in this aspect that a blade face that is tilted relative to the central axis is formed at the needle tip of the inner needle, while the outer peripheral engagement part is provided in a position corresponding to the blade face on the circumference.

Generally, the body skin is punctured by the inner and outer needles of the indwelling needle assembly in a state where the blade face of the inner needle faces the opposite side to the body skin, so that, in the indwelling needle assembly of this structure, during puncture, the outer peripheral engagement part formed outside is located on the opposite side to the body skin in the same way as the blade face of the inner needle. This can decrease the risk of lowered manipulation easiness by the outer peripheral engagement part being caught by the skin of the patient, or the like.

A fourth aspect of the present invention provides the indwelling needle assembly according to any of the first through third aspects, wherein the needle tip protector has an insertion hole receiving the inner needle being inserted through the insertion hole, and a shutter member configured to cover the insertion hole by movement of the needle tip protector to a distal end side of the inner needle.

According to the indwelling needle assembly having the structure of the present aspect, the shutter member covers the insertion hole of the inner needle in the needle tip protector moved to the distal end side of the inner needle by the extraction of the inner needle, thereby stably protecting the needle tip of the inner needle also in the axial direction. This makes it possible to more surely avoid scatter of blood etc. attached on the distal end of the inner needle and unintentional pricking due to inadvertent protrusion of the inner needle.

A fifth aspect of the present invention provides the indwelling needle assembly according to the fourth aspect, wherein the shutter member is provided integrally with the connection member.

According to the indwelling needle assembly having the structure of the present aspect, accompanying the extraction of the inner needle from the outer needle, the shutter member and the connection member are moved to the inner peripheral side of the outer needle hub, whereby the shutter member covers the insertion hole. This realizes both disconnection of the outer needle hub and the needle tip protector and obstruction of the insertion hole, so that the scatter of blood and the like during the removal of the inner needle from the outer needle can be more effectively avoided.

A sixth aspect of the present invention provides the indwelling needle assembly according to any of the first through fifth aspects, wherein a protection sleeve receiving the inner needle being inserted through the protection sleeve is assembled to the needle tip protector in a movable manner in an axial direction, and movement of the connection member to the inner peripheral side of the outer needle hub is restricted by contact of the connection member with an outer peripheral face of the protection sleeve so as to keep the connected state of the outer needle hub to the needle tip protector by the connection member, and the disconnection mechanism includes an interlocking mechanism interlocking the protection sleeve with the inner needle being extracted from the outer needle to move the protection sleeve within the needle tip protector to a proximal end side of the needle tip protector and releasing restriction against the movement of the connection member by the contact of the connection member with the protection sleeve so that the connection member is moved to the inner peripheral side so as to release the connected state.

According to the indwelling needle assembly having the structure of the present aspect, the protection sleeve, which is disposed externally about the inner needle, restricts the movement of the connection member to the inner peripheral side. Moreover, the movement of the protection sleeve to the proximal end side releases the connected state between the outer needle hub and the needle tip protector by the connection member. Thus, if a plurality of kinds of inner needles having various outer diameter dimension are used, it is not necessary to change the size or the shape for the connection member, and the like, and it is possible to use the needle tip protector of the same size.

By comparison with the case where the movement of the connection member to the inner peripheral side is restricted by contact of the connection member with the inner needle, sliding resistance during manipulation of the extraction of the inner needle from the outer needle is made smaller, so that it is possible to further improve the manipulation easiness in the extraction of the inner needle from the outer needle.

A seventh aspect of the present invention provides the indwelling needle assembly according to any of the first through sixth aspects, wherein the needle tip protector has a check window permitting a visual check of a moved position of the connection member.

According to the indwelling needle assembly having the structure of this aspect, through the check window, it is possible to visually check the movement of the connection member to the inner peripheral side, namely the connection or the disconnection between the outer needle hub and the needle tip protector by the connection member. Since it is possible to remove the inner needle from the outer needle after checking the disconnection between the outer needle hub and the needle tip protector in this way, the inner needle extraction manipulation can be performed with relief after check of secure connection of the outer needle hub and the needle tip protector, while forcible removal of the needle tip protector from the outer needle hub where the disconnection is not completed can be avoided. This makes it possible to readily improve the reliability in the work.

### EFFECT OF THE INVENTION

For the indwelling needle assembly having the structure of the present invention, the connection member connecting the outer needle hub and the needle tip protector is moved to the inner peripheral side of the outer needle hub so as to release the connection, whereby the size of the indwelling needle assembly is prevented from becoming unnecessarily large to the outer peripheral side of the outer needle hub. Consequently, the indwelling needle assembly can be handled more easily, while the manipulation easiness thereof can be improved as well.

### BRIEF DESCRIPTION OF THE DRAWINGS

FIG. 1 is a perspective view of an indwelling needle assembly as a first embodiment of the present invention.
FIG. 2 is a bottom view of the indwelling needle assembly shown in FIG. 1.
FIG. 3 is a right side view of the indwelling needle assembly shown in FIG. 1.
FIG. 4 is a cross sectional view taken along line 4-4 of FIG. 3.
FIG. 5 is a fragmentary enlarged view of a principal part of FIG. 4.
FIG. 6 is a fragmentary enlarged view of a principal part of a cross section taken along line 6-6 of FIG. 3.
FIG. 7 is a view suitable for illustrating an inside structure of the indwelling needle assembly shown in FIG. 1.
FIG. 8 is a view suitable for describing the inside structure of the indwelling needle assembly shown in FIG. 1, showing it in a perspective way through an inner needle hub and a part of a needle tip protector.
FIG. 9 is a view suitable for depicting a state of the indwelling needle assembly shown in FIG. 1 when an inner needle is extracted from the indwelling needle assembly, before operation of a disconnection mechanism, and a cross sectional view corresponding to FIG. 4.
FIG. 10 is a fragmentary enlarged view of a principal part of FIG. 9.
FIG. 11 is a view suitable for depicting a state when the inner needle has been drawn out from the indwelling needle assembly shown in FIG. 1, after the operation of the disconnection mechanism, and a cross sectional view corresponding to FIG. 9.
FIG. 12 is a view suitable for depicting the state when the inner needle has been extracted from the indwelling needle assembly shown in FIG. 1, after the operation of the disconnection mechanism, and a cross sectional view corresponding to FIG. 7.
FIGS. 13A and 13B are views suitable for illustrating a principal part of an indwelling needle assembly as a second embodiment of the present invention, which show a state before operation of a disconnection mechanism when an inner needle is extracted from the indwelling needle assembly.
FIG. 14 is an enlarged view suitable for depicting a connected section between a needle tip protector and an outer needle hub in the indwelling needle assembly shown in FIGS. 13A and 13B.
FIGS. 15A and 15B are views suitable for describing a state when the inner needle has been extracted from the indwelling needle assembly shown in FIGS. 13A and 13B, after the operation of the disconnection mechanism.
FIG. 16 is a view suitable for illustrating a principal part of an indwelling needle assembly as a third embodiment of the present invention, which shows a state before operation of a disconnection mechanism when an inner needle is extracted from the indwelling needle assembly.
FIG. 17 is a view suitable for depicting a principal part of the indwelling needle assembly shown in FIG. 16, in a perspective way through a protector main body.
FIG. 18 is a view suitable for describing a state when the inner needle has been extracted from the indwelling needle assembly shown in FIG. 16, after the operation of the disconnection mechanism.
FIGS. 19A and 19B are longitudinal cross sectional views showing a principal part of an indwelling needle assembly as another embodiment of the present invention, wherein FIG. 19A shows an initial state, while FIG. 19B shows a state after operation of a disconnection mechanism.
FIGS. 20A and 20B are longitudinal cross sectional views showing a principal part of an indwelling needle assembly as yet another embodiment of the present invention.

### EMBODIMENTS FOR CARRYING OUT THE INVENTION

In order to further specify the present invention, embodiments of the present invention will be described below in reference to the drawings.

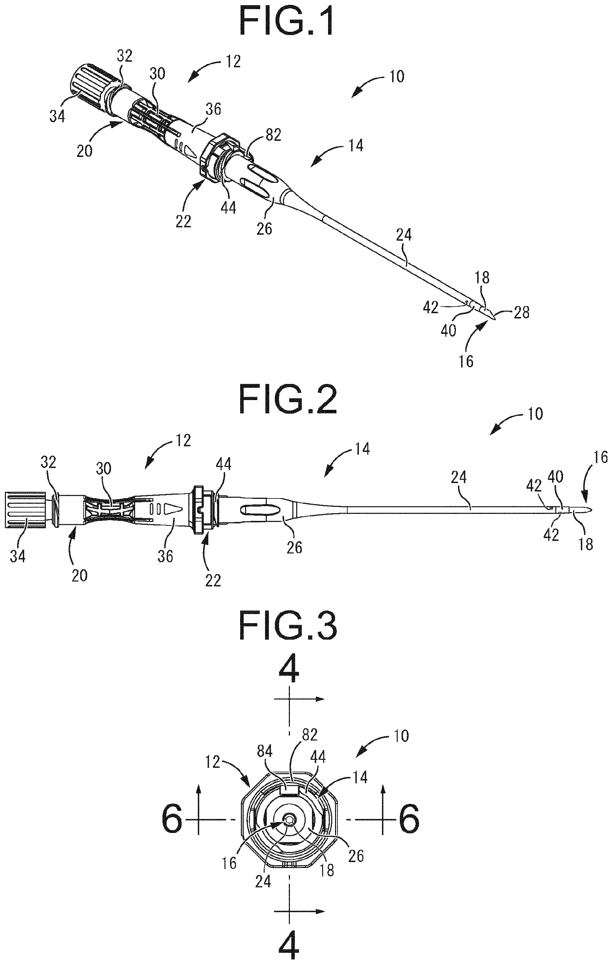

First, FIGS. 1 to 7 show an indwelling needle assembly 10 as a first embodiment of this invention. This indwelling needle assembly 10 comprises an inner needle unit 12 and an outer needle unit 14. The inner needle unit 12 includes an inner needle 18 whose distal end has a needle tip 16, and an inner needle hub 20 provided on the proximal end side of the inner needle 18. Additionally, a needle tip protector 22 is mounted to the inner needle 18, as disposed externally about the inner needle 18 movably in the needle axial direction. On the other hand, the outer needle unit 14 includes an outer needle 24, and an outer needle hub 26 provided on the proximal end side of the outer needle 24. The inner needle 18 is inserted through the outer needle unit 14 from the proximal end side. After puncture by the indwelling needle assembly 10, the inner needle 18 is drawn out from the outer needle unit 14, so that the outer needle unit 14 is detained in the skin of the patient, while the needle tip protector 22 is moved to the distal end of the inner needle 18, thereby protecting the needle tip 16 of the inner needle 18. In the descriptions hereinafter, the axial direction means the left-right direction in FIG. 2 in which the inner needle 18 and the outer needle 24 extend. The distal end side means the right side in FIG. 2, which is the side of the needle tip 16 of the inner needle 18, while the proximal end side means the left side in FIG. 2, which is the side on which the user manipulates the indwelling needle assembly.

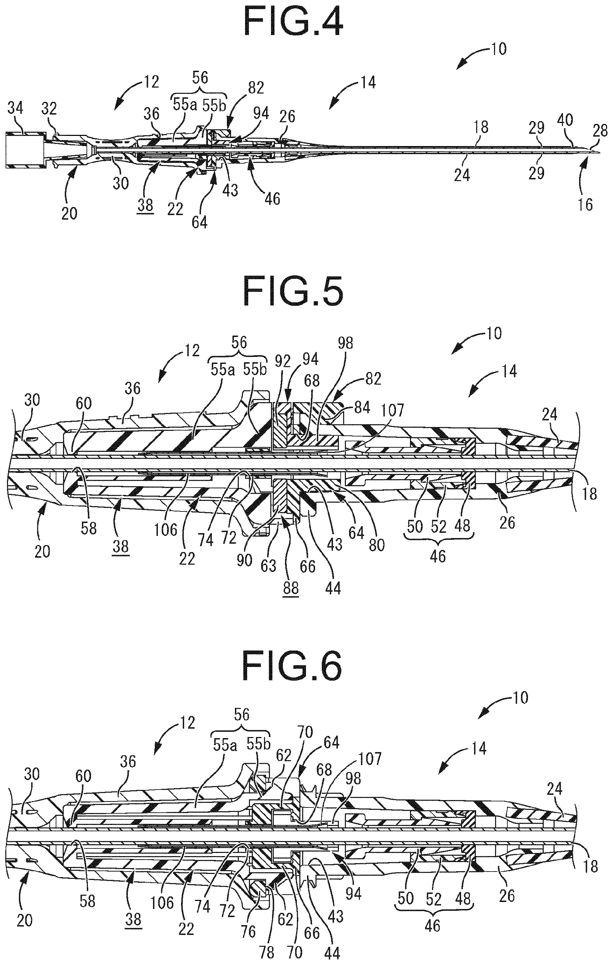

More specifically, in the inner needle unit 12, the inner needle 18 is a hollow needle formed of known material such as stainless steel, aluminum, titanium, and alloy of some of them, for example. At the needle tip 16 of the inner needle 18, a blade face 28 is formed with a sharp shape enabled to easily puncture the living body expanding in a direction tilted relative to the axial direction. At the distal end of the inner needle 18, large-diameter parts 29 are formed to have a slightly larger outer diameter dimension. In the present embodiment, on both sides of one diametrical direction (both sides of the up-down direction in FIG. 4), a pair of large-diameter parts 29, 29 are formed. Note that the inner needle 18 may be a solid needle.

The inner needle hub 20, which is provided on the proximal end side of the inner needle 18, is made of rigid synthetic resin material such as polypropylene, in a long roughly-cylindrical shape as a whole. The middle portion in the length direction of the inner needle hub 20 is an inner needle fixation part 30 whose outer diameter is narrowed in a constricted shape. The proximal end of the inner needle 18 is fixed on the inner needle fixation part 30, while piercing through it.

The opening part on the proximal end side of the inner needle fixation part 30 in the inner needle hub 20 is a juncture part 32 whose outer peripheral face is provided with a screw thread. To the juncture part 32, a port member 34 is fixed for connection with the outside tube passage. Specifically, a syringe or the outside tube passage is linked to the port member 34, whereby the outside tube passage communicates with the lumen of the inner needle 18, in the inner needle hub 20. A ventilation filter is provided inside the port member 34, as needed.

The opening part on the distal end side of the inner needle fixation part 30 in the inner needle hub 20 is a cover tubular part 36 extending in the axial direction with a large-diameter cylindrical shape. Thus, on the radially inner side of the cover tubular part 36, a housing void 38 is formed opening to the distal end side.

On the other hand, for the outer needle unit 14, the outer needle 24 has a tubular shape, and it is made of material having moderate flexibility, e.g., various soft resins such as ethylene tetrafluoroethylene copolymer (ETFE), polyurethane, and polyether nylon resin. On the distal end of the outer needle 24, a tapered outer peripheral face 40 that is tapered is formed, thereby alleviating puncture resistance in relation to the living body. In the peripheral wall of the distal end of the outer needle 24, a passage hole 42 is provided, thereby improving circulation efficiency of the fluid for the outer needle 24.

The outer needle hub 26 provided on the proximal end side of the outer needle 24 is formed of rigid synthetic resin material such as polypropylene, in a long generally-cylindrical shape as a whole. The distal end side of the outer needle hub 26 has a tapered outer peripheral face whose diameter becomes gradually smaller as it approaches the outer peripheral face of the outer needle 24. Meanwhile, in a proximal end side opening part 43 of the outer needle hub 26, a lock part 44 is formed with a screw thread on its outer peripheral face. After the outer needle unit 14 is detained in the skin of the patient, a luer lock syringe etc. is connected to the proximal end side opening part 43 of the outer needle hub 26.

The inner diameter of the outer needle hub 26 is larger than the outer diameter of the inner needle 18, which is inserted in the outer needle hub 26, and a hemostasis valve mechanism 46 is stored inside the outer needle hub 26. The structure of the hemostasis valve mechanism 46 is not under any limitation. In this embodiment, the hemostasis valve mechanism 46 comprises a hemostasis valve main unit 48, a pusher 50, and a pusher guide 52.

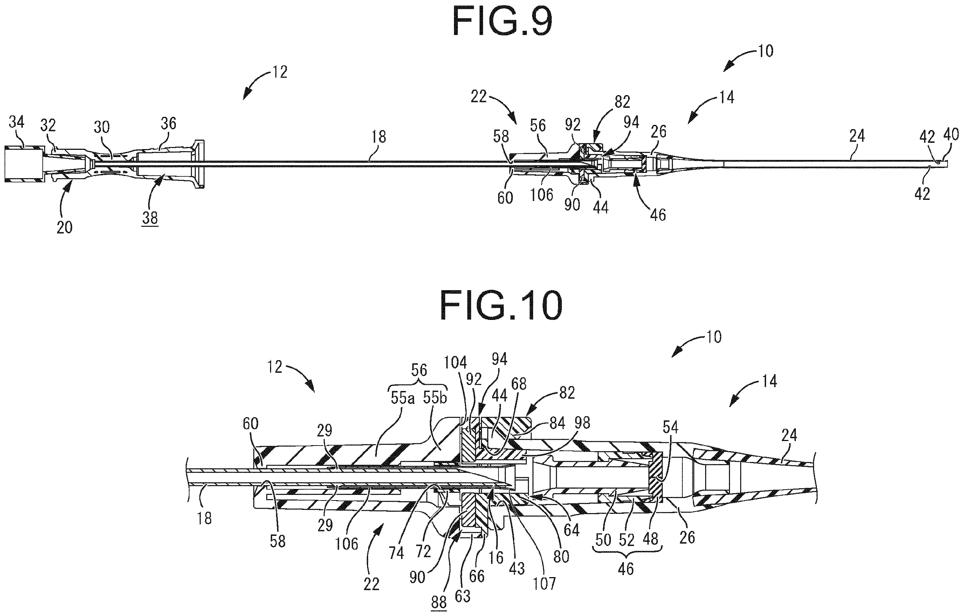

The hemostasis valve main unit 48 is a disk valve having a slit 54 (see FIG. 10) at its center, and it is formed using a rubber elastic body or an elastomer. The pusher 50 has a nearly tubular shape as a whole, wherein its inner diameter dimension is larger than the outer diameter dimension of the inner needle 18. The pusher guide 52 has a substantially tubular shape as a whole, and the pusher guide 52 is disposed radially between the pusher 50 and the outer needle hub 26. This hemostasis valve main unit 48 is attached inside the outer needle hub 26. The pusher 50 and the pusher guide 52 are positioned on the proximal end side of the hemostasis valve main unit 48, and the pusher 50 is allowed to move in the axial direction while sliding along the pusher guide 52. In this embodiment, an annular concave groove is provided at the outer peripheral part on the proximal end side end face of the hemostasis valve main unit 48, while the distal end side end face of the pusher guide 52 protrudes in a tubular shape, and the protruding portion enters the annular concave groove of the hemostasis valve main unit 48.

After the outer needle unit 14 is detained in the skin of the patient, the syringe etc. is inserted via the proximal end side opening part 43 of the outer needle hub 26, whereby the distal end of the syringe pushes the pusher 50 to the distal end side, thus opening the slit 54 of the hemostasis valve main unit 48. Consequently, a liquid medicine or the like in the syringe is injected into the blood vessel of the patient through the outer needle unit 14. After the injection, the syringe is extracted from the outer needle unit 14, whereby the slit 54 is rapidly closed by elastic recovery action of the hemostasis valve main unit 48.

In the inner needle unit 12, the needle tip protector 22 is attached to the inner needle 18, as disposed externally about the inner needle 18.

This needle tip protector 22 comprises a protector main body 56 that includes a peripheral wall in a stepped cylindrical shape constituted by a small-diameter tubular part 55a on the proximal end side and a large-diameter tubular part 55b on the distal end side. At the opening part on the side of the small-diameter tubular part 55a of the protector main body 56, a bottom wall 60 is provided while having a proximal end side needle insertion hole 58 at its center. On the other hand, at the opening end on the side of the large-diameter tubular part 55b, detaining claws 62, 62 are provided projecting radially outward on both sides of one diametrical direction (both sides of the up-down direction in FIG. 6). Moreover, in the distal end side end face of the large-diameter tubular part 55b, on both sides of a direction orthogonal to the facing direction of the detaining claws 62, 62 (both sides of the up-down direction in FIG. 5), outer peripheral collar parts 63 are formed. The outer peripheral collar part 63 protrudes with a prescribed height from the outer peripheral edge of the end face to the distal end side, while curving in the circumferential direction. A cover member 64 is assembled to the distal end side opening part of the protector main body 56.

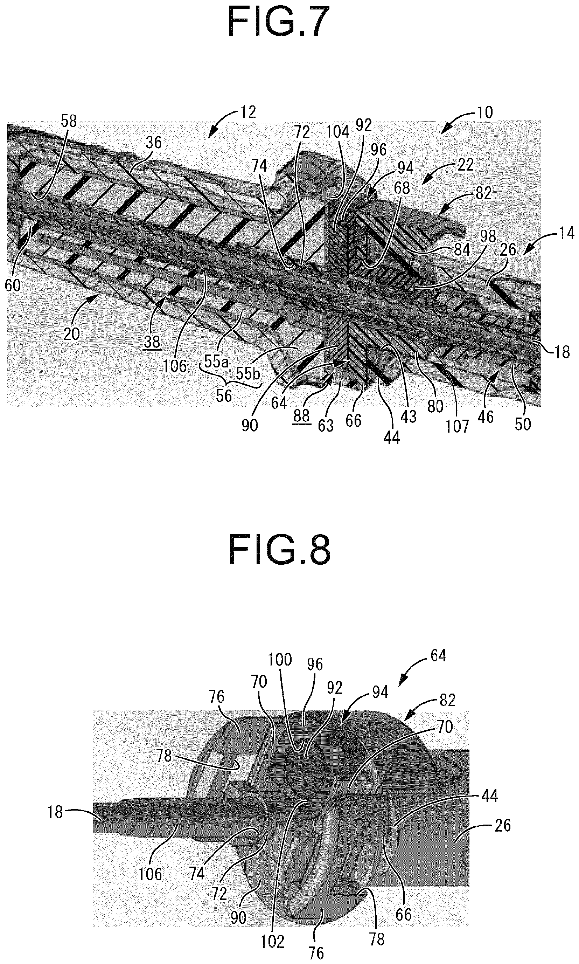

As FIG. 8 shows, the cover member 64 has a lid part 66 of about circular disk shape. At the center of the lid part 66, a central hole 68 is provided so that the inner needle 18 is inserted in the central hole 68. The outer diameter dimension of the lid part 66 is nearly equal to the outer diameter dimension of the large-diameter tubular part 55b of the protector main body 56, and larger than the outer diameter dimension of the lock part 44 provided at the proximal end side opening part 43 of the outer needle hub 26.

The central hole 68 of the lid part 66 is in a generally oval shape expanding outward in a portion on the circumference (a part on the upper side in FIG. 5), as viewed in the axial direction of the lid part 66. On the end face of the lid part 66 on the left side of FIG. 6, which is the proximal end side, a pair of guide walls 70, 70 are provided protruding in the axial direction along the central hole 68 from both sides of the width direction (the both sides of the up-down direction in FIG. 6) across the central hole 68. Furthermore, a tube-shaped holding tubular part 72 extending in the axial direction is formed at the protruding ends of the guide walls 70, 70. The inner diameter dimension of the holding tubular part 72 is slightly larger than the outer diameter dimension of the inner needle 18, and the inner hole of the holding tubular part 72 is a distal end side needle insertion hole 74 working as an insertion hole through which the inner needle 18 is inserted. The protrusion height dimension for the guide walls 70, 70 is substantially the same as or slightly larger than the axial dimension for a fixation member 90 and a shutter member 92 described later, and it is slightly smaller than the axial dimension of the outer peripheral collar part 63.

At the outer peripheral edge of the lid part 66, a pair of external fit parts 76, 76 are provided radially outside the guide walls 70, 70, so as to protrude toward the proximal end side in the axial direction. At the circumferentially central portions of the external fit parts 76, 76, detaining windows 78, 78 are formed opening to the radial outside. In size, the detaining windows 78, 78 correspond to the detaining claws 62, 62 provided in the protector main body 56.

In the lid part 66, an inside fitting part 80 is formed protruding from the opening peripheral edge of the central hole 68 toward the distal end side (the right side in FIG. 5), at a position on the side opposed to the side to which the aforesaid central hole 68 expands (the lower side in FIG. 5). The inside fitting part 80 is a roughly half tubular body. The outer diameter dimension of the inside fitting part 80 (an outer diameter dimension in assumption of a whole cylindrical shape) is nearly equal to the inner diameter dimension of the proximal end side opening part 43 of the outer needle hub 26. Meanwhile, the inner diameter dimension of the inside fitting part 80 (an inner diameter dimension in the assumption of the whole cylindrical shape) is larger than the outer diameter dimension of the inner needle 18. This inside fitting part 80 is formed with a protrusion dimension with which the inside fitting part 80 does not touch the proximal end face of the pusher 50 in the assembled state of the inner needle unit 12 and the outer needle unit 14.

At the outer peripheral edge of the lid part 66, an engagement piece 82 is provided extending out to the distal end side in the axial direction, at a portion on the circumference (the part on the upper side in FIG. 5). The engagement piece 82 is a roughly plate-shaped body that takes a substantially rectangular shape in a top plan view from the upper side of FIG. 5 and curves in the circumferential direction in an elevational view, taken from the right side of FIG. 5. Additionally, a claw 84 is provided projecting radially inward, at the distal end of the engagement piece 82.

The detaining claws 62, 62 of the protector main body 56 enter the detaining windows 78, 78 of the cover member 64 so as to be engaged therein. By so doing, the distal end side end face of the outer peripheral collar part 63 provided in the protector main body 56 is abutted against the proximal end side end face of the lid part 66. As a result, the distal end side opening part of the protector main body 56 is covered by the cover member 64, so that the cover member 64 is assembled to the protector main body 56. Then, the large-diameter tubular part 55b of the protector main body 56 and the lid part 66 are disposed with a prescribed distance in between in the axial direction, and an inner space 88 is formed in the axial space between them, which is the radial inside of the outer peripheral collar part 63. The pair of guide walls 70, 70 extend out within the inner space 88.

Here, in the inner space 88, the fixation member 90 securely attached to the cover member 64 and the shutter member 92 that is movable in the diametrical direction within the inner space 88 are disposed and stored. The fixation member 90 and the shutter member 92 have substantially the same disk shape. In the initial state shown in FIG. 4, and the like, the fixation member 90 and the shutter member 92 are located on both sides of one diametrical direction (the up-down direction in FIG. 4) across the inner needle 18. This fixation member 90 is fixed to the proximal end side of the inside fitting part 80, namely, the proximal end side end face of the cover member 64, in the lower side in FIG. 4. On the other hand, the shutter member 92 is located on the opposite side in the diametrical direction across the inner needle 18 (in the upper side in FIG. 4).

In the present embodiment, the fixation member 90 is constituted by a ferromagnetic material like a metal, while the shutter member 92 is constituted by a permanent magnet. Thus, the shutter member 92 is attracted in relation to the fixation member 90, so that the shutter member 92 is allowed to move in the diametrical direction in the inner space 88. In other words, on the shutter member 92, an urging force of a direction of approaching the fixation member 90 is always exerted. It is also possible that the fixation member is a permanent magnet, while the shutter member is made of ferromagnetic material. Alternatively, it is possible that both the fixation member and the shutter member are formed of permanent magnet such that an attractive force is exerted between them.

To the shutter member 92, a slide member 94 that is movable integrally with the shutter member 92 is fixed. The slide member 94 is assembled such that the slide member 94 is movable in the diametrical direction relative to the cover member 64 while being guided by the guide walls 70, 70. This slide member 94 is preferably formed of non-magnetic material like synthetic resin, and includes a base part 96 on the proximal end side, and a distal end protruding part 98 that protrudes from the distal end side end face of the base part 96.

The base part 96 has a block shape whose entire transverse face is about rectangular shape with round corners. At the center of the proximal end side end face of the base part 96, a circular storing recess 100 is formed. The shutter member 92 is fixed on and stored in the storing recess 100. A notch 102 is formed on the side that faces the fixation member 90 in the outer peripheral wall of the storing recess 100, whereby the shutter member 92 is exposed to the inner peripheral side via the notch 102.

Meanwhile, in the distal end side end face of the base part 96, the distal end protruding part 98 is formed protruding from the inner peripheral edge thereof to the distal end side. This distal end protruding part 98 has a curved plate shape curved in the circumferential direction corresponding to the shape of the inner peripheral face of the outer needle hub 26 and takes a roughly rectangular shape in a top plan view from the upper side of FIG. 5. When the inner needle unit 12 and the outer needle unit 14 are assembled, the distal end protruding part 98 extends out to about the same axial position as that of the inside fitting part 80 so that the distal end protruding part 98 does not touch the pusher 50.

The base part 96 is positioned between the opposite faces of the guide walls 70, 70 provided on the proximal end side end face of the lid part 66, while the distal end protruding part 98 extends further to the distal end side beyond the lid part 66 via the central hole 68 of the lid part 66. Specifically, the base part 96 of the slide member 94 is housed in the inner space 88, while the distal end protruding part 98 of the slide member 94 extends out further to the distal end side than the inner space 88 via the central hole 68. As a result, the base part 96 is made movable while being guided by the both guide walls 70, 70, while, accompanying the movement of the base part 96, the distal end protruding part 98 is moved in the central hole 68, so that the whole slide member 94 is assembled as movable in the diametrical direction relative to the cover member 64.

As has been described above, the fixation member 90, the shutter member 92, and the slide member 94 are stored in the inner space 88 defined by the protector main body 56 and the cover member 64, thereby constituting the needle tip protector 22 of this embodiment. In the present embodiment, an opening window 104 serving as a check window is formed at the central portion in the circumferential direction of the outer peripheral collar part 63 on the upper side in FIG. 4. The outer peripheral face of the base part 96 of the slide member 94 is exposed to the outside space via the opening window 104. Especially, in the present embodiment, the outer peripheral face of the base part 96 and the outer peripheral face of the engagement piece 82 are positioned on the same curved face.

The inner needle 18 is inserted through the aforesaid needle tip protector 22 from the proximal end side, thus constituting the inner needle unit 12 of this embodiment. Specifically, in the initial state shown in FIG. 1, and the like, the protector main body 56 is stored within the housing void 38 of the cover tubular part 36. In addition to that, from the proximal end side, the inner needle 18 is inserted through the proximal end side needle insertion hole 58, the distal end side needle insertion hole 74, and the central hole 68, in this order, so as to extend out to the distal end side. Then, in the inner space 88, the shutter member 92 is located on one side of the inner needle 18 (the upper side in FIG. 4), while the fixation member 90 is located on the other side of the inner needle 18 (the lower side in FIG. 4).

In the present embodiment, a protection sleeve 106 is attached to the inner needle 18, as disposed externally about it. The protection sleeve 106 is a tubular member extending in the axial direction as a whole, and is movable in the axial direction relative to the inner needle 18. The axial dimension of the protection sleeve 106 is smaller than an axial dimension from the distal end face of the bottom wall 60 of the protector main body 56 to the proximal end side end face of the shutter member 92.

The inner diameter dimension of the axially middle portion of the protection sleeve 106 is larger than the outer diameter dimension for the large-diameter parts 29, 29 of the inner needle 18, while the inner diameter dimension of the axially proximal end thereof is reduced to be smaller than the outer diameter dimension for the large-diameter parts 29, 29. Besides, the outer diameter dimension of the axially proximal end of the protection sleeve 106 is larger than the inner diameter dimension of the proximal end side needle insertion hole 58, while the outer diameter dimension of the axially middle portion thereof is smaller than the inner diameter dimension for the distal end side needle insertion hole 74 and the central hole 68. Specifically, the movement of the protection sleeve 106 relative to the inner needle 18 to the distal end side is restricted by engagement between the large-diameter parts 29, 29 and the proximal end of the protection sleeve 106. Additionally, the movement of the protection sleeve 106 relative to the protector main body 56 to the proximal end side is restricted by engagement between the large-diameter parts 29, 29 and the bottom wall 60 of the protector main body 56.

Since the protection sleeve 106 is provided, the outer peripheral face of the protection sleeve 106 is abutted on the slide member 94, thereby restricting the movement of the shutter member 92 and the slide member 94 to the inner peripheral side in the initial state.

The distal end of the protection sleeve 106 of this embodiment is a flared part 107 whose outline dimension is slightly expanded in diameter, and its diameter dimension is made larger than the inner diameter dimension of the pusher 50 and made slightly larger than the inner diameter dimension of the distal end side needle insertion hole 74. As a result, in the initial state, the distal end of the protection sleeve 106 is located axially between the holding tubular part 72 and the pusher 50. In other words, the protection sleeve 106, through which the inner needle 18 is inserted, is assembled axially movably in a prescribed position of the needle tip protector 22.

The inner needle 18 of the inner needle unit 12 is inserted through the outer needle unit 14, thus constituting the indwelling needle assembly 10 of this embodiment. Specifically, the inner needle 18 is inserted through the pusher 50, the hemostasis valve main unit 48, and the outer needle 24, so that the needle tip 16 of the inner needle 18 is located further to the distal end side than the outer needle 24. Then, the inside fitting part 80 of the cover member 64 touches the inner peripheral face of the outer needle hub 26. In addition to that, the engagement piece 82 extends out to the outside of the outer needle hub 26, and the claw 84 of the engagement piece 82 is engaged in the lock part 44 provided on the outer peripheral face of the proximal end side opening part 43 of the outer needle hub 26. Also, the distal end protruding part 98 of the slide member 94 extends out from the needle tip protector 22 to the inside of the outer needle hub 26 while being abutted against the inner peripheral face of the outer needle hub 26. The distal end protruding part 98 and the claw 84 of the engagement piece 82 provided in the needle tip protector 22 clamp and hold the peripheral wall of the outer needle hub 26 in the radial direction. This restricts relative movement between the needle tip protector 22 and the outer needle hub 26.

Specifically, in this embodiment, the connection mechanism is constituted to connect the distal end side of the needle tip protector 22 and the proximal end side of the outer needle hub 26 by the claw 84 of the engagement piece 82 and the distal end protruding part 98 of the slide member 94 clamping the peripheral wall of the outer needle hub 26. The slide member 94 is a connection member keeping the connected state in this connection mechanism. Moreover, the engagement piece 82 and the claw 84 constitute the outer peripheral engagement part. The outer peripheral engagement part is abutted on the outer face of the outer needle hub 26 as the peripheral wall of the outer needle hub 26 is clamped between the outer peripheral engagement part and the slide member 94. In this embodiment, the protection sleeve 106 is provided to restrict the movement of the slide member 94 to the inner peripheral side, thus keeping the connected state between the needle tip protector 22 and the outer needle hub 26.

The indwelling needle assembly 10 of the present embodiment having the aforesaid structure is generally used to pierce the skin of the patient, with the blade face 28 of the inner needle 18 facing the upper side. While keeping the state where the skin of the patient is punctured by the indwelling needle assembly 10, the inner needle unit 12 is drawn out of the outer needle unit 14, so that the outer needle unit 14 is detained in the skin of the patient.

In this embodiment, the engagement piece 82, which is engaged in the outer peripheral face of the outer needle hub 26, is formed in the position corresponding to the blade face 28 of the inner needle 18 on the circumference (the upper side in FIG. 4). Thus, when the blade face 28 faces the upper side, the engagement piece 82 is located at the upper side of the indwelling needle assembly 10. This avoids the risk of sticking of the engagement piece 82 to the skin of the patient, and the like, thereby improving the manipulation easiness.

There will be hereafter explained manipulation when extracting the inner needle unit 12 from the outer needle unit 14, while referring to FIGS. 9 to 12.

First, with the state where the skin of the patient is punctured by the indwelling needle assembly 10, as FIGS. 9 and 10 show, the inner needle unit 12 is moved to the proximal end side relative to the outer needle unit 14. By so doing, the inner needle 18 of the inner needle unit 12 is extracted from the outer needle 24. Then, the claw 84 of the engagement piece 82 is engaged in the lock part 44, while the peripheral wall of the outer needle hub 26 is clamped radially between the claw 84 and the distal end protruding part 98 provided in the slide member 94. Consequently, the needle tip protector 22 mounted on the inner needle 18 as disposed externally about it does not move to the proximal end side, so that the connected state between the needle tip protector 22 and the outer needle hub 26 is maintained.

By extraction of the inner needle 18 from the hemostasis valve main unit 48, the slit 54 of the hemostasis valve main unit 48 is closed yielding to the elasticity of the hemostasis valve main unit 48. This makes it possible to avoid reverse flow of the blood.

The inner needle 18 moves to the proximal end side relative to the protection sleeve 106, whereby the large-diameter parts 29, 29 of the inner needle 18 are caught and engaged in the proximal end of the protection sleeve 106. In this state, the needle tip 16 of the inner needle 18 is preferably stored in the protection sleeve 106. The flared part 107 is provided at the distal end of the protection sleeve 106, thereby making it easy for the large-diameter parts 29, 29 of the inner needle 18 to enter the inside of the protection sleeve 106.

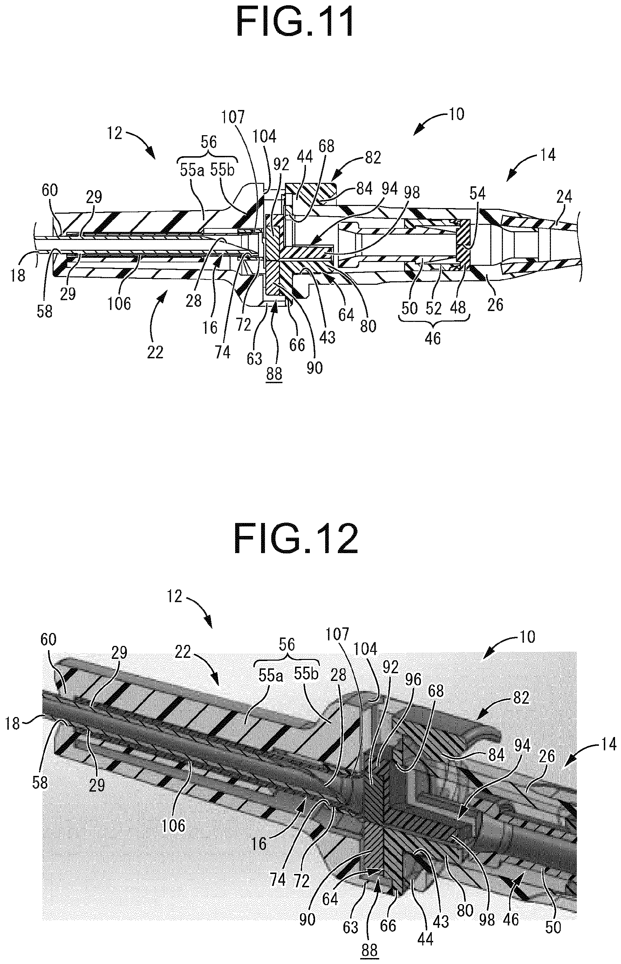

With the protection sleeve 106 engaged in the inner needle 18, the inner needle 18 is further drawn out to the proximal end side, whereby the protection sleeve 106 is moved to the proximal end side relative to the needle tip protector 22, accompanying the inner needle 18, as FIGS. 11 and 12 show. Specifically, in this embodiment, the large-diameter parts 29, 29 of the inner needle 18 are engaged in the proximal end of the protection sleeve 106, thus constituting the interlocking mechanism by which the inner needle 18 and the protection sleeve 106 move with correlation. The movement of the inner needle 18 and the protection sleeve 106 to the proximal end side is restricted by contact between the bottom wall 60 of the protector main body 56 and the proximal end of the protection sleeve 106. In this state, the protection sleeve 106 is stored inside the protector main body 56. In other words, the needle tip protector 22 moves to the distal end side beyond the needle tip 16 of the inner needle 18.

The movement of the protection sleeve 106 described above to the proximal end side relative to the needle tip protector 22 releases the contact between the protection sleeve 106 and the slide member 94. The slide member 94 moves integrally with the shutter member 92 to the inner peripheral side of the outer needle hub 26, yielding to the attractive force between the shutter member 92 and the fixation member 90. Specifically, in this embodiment, the urging means exerting the urging force of the direction to move the slide member 94 to the inner peripheral side of the outer needle hub 26 is constituted by including the shutter member 92. The shutter member 92 and the fixation member 90 get into contact, thereby restricting the movement of the shutter member 92 and the slide member 94 to the inner peripheral side. Then, the shutter member 92 is located on the distal end side of the holding tubular part 72, thereby covering the distal end side opening part of the distal end side needle insertion hole 74. Therefore, by the shutter member 92, the needle tip 16 of the inner needle 18 is axially covered and protected.

The slide member 94 moves to the inner peripheral side of the outer needle hub 26, so that the distal end protruding part 98 in contact with the inner peripheral face of the outer needle hub 26 moves to a position separated from the inner peripheral face of the outer needle hub 26 to the inner peripheral side. This releases the clamping of the peripheral wall of the outer needle hub 26 by the claw 84 of the engagement piece 82 and the distal end protruding part 98. By the movement of this distal end protruding part 98 to the inner peripheral side of the outer needle hub 26, a gap is formed radially between the claw 84 and the distal end protruding part 98, thus making it possible to displace the inner needle unit 12 relative to the outer needle unit 14 to one side of one diametrical direction (the upper side in FIG. 11). By the relative displacement of the inner needle unit 12 to the side, the engagement between the claw 84 of the engagement piece 82 and the lock part 44 of the outer needle hub 26 is released, whereby the inner needle unit 12 is removed from the outer needle unit 14.

Specifically, the disconnection mechanism is constituted such that, accompanying the extraction of the inner needle 18 from the outer needle 24, the slide member 94 (the connection member) is moved to the inner peripheral side of the outer needle hub 26, thus disconnecting the needle tip protector 22 and the outer needle hub 26. Particularly in this embodiment, the protection sleeve 106 is disposed externally about the inner needle 18, and the interlocking mechanism with which the inner needle 18 and the protection sleeve 106 move while interlocking is provided. Therefore, the disconnection mechanism is constituted by including the interlocking mechanism.

In the present embodiment, the opening window 104 is formed at a part of the outer peripheral collar part 63, and the base part 96 of the slide member 94 is exposed to the outside space via the opening window 104. Thus, via the opening window 104, it is possible to visually check the position of the slide member 94, or the difference of the position of the slide member 94 moved to the inner peripheral side, from the initial position with which the outer peripheral end of the slide member 94 is in substantially the same position as that of the outer peripheral face of the outer peripheral collar part 63.

For the indwelling needle assembly 10 of this embodiment having the above-mentioned shape, when the inner needle unit 12 is removed from the outer needle unit 14, the slide member 94 moves to the inner peripheral side of the outer needle hub 26, thereby releasing the connection between the needle tip protector 22 and the outer needle hub 26. This prevents the enlargement of the indwelling needle assembly 10, so that it is possible to remove the inner needle unit 12 from the outer needle unit 14 with good manipulation easiness.

Especially, the peripheral wall of the outer needle hub 26 is clamped by the claw 84 of the engagement piece 82 and the distal end protruding part 98 of the slide member 94, thereby realizing the connection between the needle tip protector 22 and the outer needle hub 26. Meanwhile, the distal end protruding part 98 is moved to the inner peripheral side, thereby releasing this connection. Specifically, the member for keeping the connected state is moved, thereby releasing the connected state, so that the operation of the connection mechanism and the disconnection mechanism can be stable.

In the present embodiment, the shutter member 92 moves to the inner peripheral side, thereby making it possible to cover the needle tip 16 of the inner needle 18 in the axial direction. Therefore, after removal of the inner needle unit 12, it is possible to more securely avoid scattering of the blood, or the like put on the needle tip 16 of the inner needle 18 and inadvertent pricking by protrusion of the inner needle 18. Particularly, since the shutter member 92 and the slide member 94 can move integrally, it is possible to realize disconnection operation for the outer needle hub 26 and the needle tip protector 22 and protection operation for the needle tip 16 of the inner needle 18 at the same time. This makes it possible to remove the inner needle unit 12 from the outer needle unit 14 more safely.

Besides, in this embodiment, the shutter member 92 is formed of permanent magnet, while the fixation member 90 is formed of ferromagnetic material. Consequently, the shutter member 92 and the slide member 94 always receive the urging force to the inner peripheral side of the outer needle hub 26. Therefore, by the extraction of the inner needle 18 to the proximal end side, the shutter member 92 and the slide member 94 can move to the inner peripheral side automatically, and immediately.

Moreover, in the present embodiment, the protection sleeve 106 is mounted on the inner needle 18, as disposed externally about it. In the initial state, the contact of the slide member 94 with the protection sleeve 106 restricts the movement of the shutter member 92 and the slide member 94 to the inner peripheral side. Specifically, in the extraction of the inner needle 18, the inner needle 18 does not slide directly on the shutter member 92 and the slide member 94, thus alleviating sliding resistance in the extraction of the inner needle 18, so that extraction manipulation can be made with good manipulation easiness.

Use of the protection sleeve 106 makes it possible to use the needle tip protector 22 of the same size, even if a plurality of kinds of inner needles 18 with various outer diameter dimensions are adopted.

In this embodiment, it is possible to visually check the movement of the slide member 94 to the inner peripheral side via the opening window 104. Thus, it is possible to release the engagement between the claw 84 of the engagement piece 82 and the lock part 44 of the outer needle hub 26 after the check of the movement of the slide member 94 to the inner peripheral side, thereby removing the inner needle unit 12 from the outer needle unit 14. This can reduce the risk of doing removal manipulation of the inner needle unit 12 from the outer needle unit 14 before the connection between the needle tip protector 22 and the outer needle hub 26 is sufficiently released.

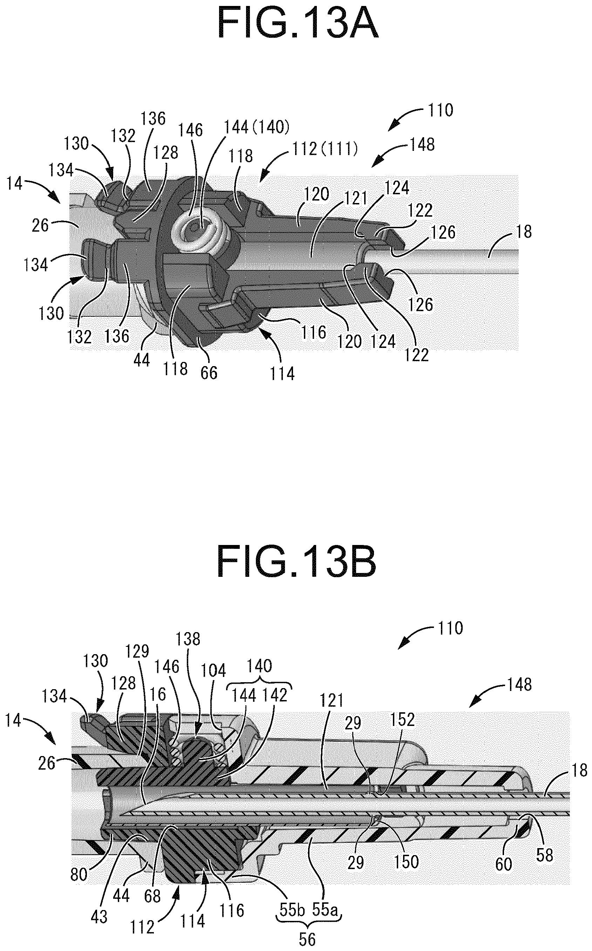

Next, FIGS. 13A and 13B show a principal part of an indwelling needle assembly 110 as a second embodiment of the present invention. In this embodiment, the cover member, the connection member, and the urging means, which urges the connection member to the inner peripheral side of the outer needle hub 26, in a needle tip protector 111 are different from those of the first embodiment. FIGS. 13A and 13B show the same state as the state of FIG. 10 of the first embodiment, made by the extraction of the inner needle 18. FIG. 13A is a perspective view showing the connected section between the needle tip protector 111 and the outer needle hub 26 as enlarged, through the protector main body 56. On the other hand, FIG. 13B is a longitudinal cross sectional view showing the state of FIG. 13A with the protector main body 56.

In the descriptions hereinafter, for substantially the same members and parts as those of the above-referred embodiment, detailed explanation will be omitted by giving the same code numbers as those of the aforesaid embodiment in the drawings. About not-shown sections, since the same structure as that of the aforesaid embodiment can be adopted, detailed description will be omitted. Additionally, in the embodiment shown in FIGS. 13A, 13B, and 15-18 below, the distal end side is shown as the left side in the drawings, while the proximal end side is shown as the right side in the drawings, differently from the above-mentioned embodiment.

Specifically, in a cover member 112 of this embodiment, a guide wall 114 of about "U" character shape having an opening at a part on the periphery is provided on the proximal end side of the lid part 66. That is, a curved wall 116 that is the bottom wall constituting the "U" character shape, and guide side walls 118, 118 that extend out nearly in a straight line from the both peripheral ends of the curved wall 116 are provided. From these guide side walls 118, 118, retaining arms 120, 120 extend out toward the proximal end side. The distance between the opposed faces of the retaining arms 120, 120 is slightly larger than the outer diameter dimension of a protection sleeve 121 of this embodiment. The axial dimension for the retaining arms 120, 120 is smaller than the axial dimension of the protection sleeve 121.

At the protruding distal ends of these retaining arms 120, 120 (the proximal ends in the needle axial direction), stopper claws 122, 122 are provided protruding to the inner peripheral side. The distance between the opposed faces of the stopper claws 122, 122 is larger than the outer diameter dimension of the inner needle 18, and smaller than the outer diameter dimension of the protection sleeve 121.

Apart of the stopper claw 122 on the proximal end side of the retaining arm 120 (the distal end side in the needle axial direction) has a stopper face constituted by an incline 124. The incline 124 is inclined toward the distal end side of the retaining arm 120 as it goes radially inward. In the inner peripheral faces that are mutually opposed in the stopper claws 122, 122, concave grooves 126, 126 are formed with curved bottom faces corresponding to the shape of the outer peripheral face of the protection sleeve 121.

Meanwhile, in the outer peripheral edge of the lid part 66, at a part on the circumference, which is at the opening side on the periphery of the guide wall 114 of about "U" character shape (the opposite side to the curved wall 116 across the inner needle 18), a positioning protrusion 128 is provided protruding to the distal end side. This positioning protrusion 128 has a tapered shape wherein the width of the distal end is narrow. On both sides of the positioning protrusion 128 in the circumferential direction, a pair of engagement pieces 130, 130 are provided extending out from the outer peripheral edge of the lid part 66, to the same side as the positioning protrusion 128. The positioning protrusion 128 and each engagement piece 130 are spaced away from each other in the circumferential direction.

At an axially middle portion of each engagement piece 130, a claw 132 is provided projecting to the inner peripheral side in a bent shape like inclined "L" character shape. The dimension in the protrusion direction of each engagement piece 130 from the proximal end part thereof on the side of the lid part 66 up to the claw 132 is roughly the same as the axial dimension of the lock part 44 in the outer needle hub 26. Consequently, the engagement piece 130 can be engaged in the lock part 44 in a state the lock part 44 is held in the axial direction between the engagement piece 130 and the lid part 66.

The cover member 112 is superposed to the distal end side opening part of the protector main body 56, and they are fixed to each other, so that an inner space 138 is formed as enclosed by the cover member 112 and the protector main body 56. Specifically, the inner space 138 is constituted by an inside space of the guide wall 114 of about "U" character shape. The inner space 138 opens to the distal end side via the central hole 68, while it communicates with the outside space via the opening window 104 provided in the protector main body 56.

In the inner space 138, a shutter member 140 serving as a connection member is housed. The shutter member 140 comprises a base part 142 of about plate shape, and a support projection 144 of substantially cylindrical shape projecting from the base part 142. This base part 140 is located in the inner space 138 between the both guide side walls 118, 118. Then, the distal end side of the base part 142 protrudes to the distal end side beyond the cover member 112 via the central hole 68. Meanwhile, the support projection 144 projecting from the base part 142 extends in the inner space 138 to the side of the opening window 104 so that it can be visually checked through the opening window 104 from the outside.

A coil spring 146 is attached to the support projection 144 as the coil spring 146 is disposed externally about the support projection 144. The coil spring 146 is stored in the inner space 138 so that an end thereof in the length direction, which is the extension/contraction direction, is abutted from inside against the opening peripheral edge of the opening window 104, while the other end in the length direction is abutted against the base part 142.

As has been described above, the needle tip protector 111 of this embodiment is constituted by mutually fixing the protector main body 56 and the cover member 112, which define the inner space 138 containing the shutter member 140 and the coil spring 146. The fixation of the protector main body 56 and the cover member 112 can be made through engagement using concave and convex shapes like the aforesaid embodiment, or various well-known fixation means, e.g., adhesion, welding, and press-fitting as employed. By inserting the inner needle 18 through this needle tip protector 111, an inner needle unit 148 of the present embodiment is constituted.

The protection sleeve 121 is attached to the inner needle 18 of the present embodiment in a state where the protection sleeve 121 is disposed externally about it. The peripheral edge of the protection sleeve 121 of this embodiment on the proximal end side in the axial direction projects to the inner peripheral side, thereby forming an annular inside projection 150. The inner hole of this inside projection 150 is a passage hole 152 through which the inner needle 18 is inserted. The diameter dimension of the passage hole 152 is smaller than the outer diameter dimension for the large-diameter parts 29, 29 of the inner needle 18, while it is slightly larger than the outer diameter dimension for the parts other than the large-diameter parts 29, 29 of the inner needle 18.

Here, for the inner needle unit 148 before operation of the disconnection mechanism shown in FIGS. 13A and 13B, the distance between the opposite faces of the stopper claws 122, 122 of the retaining arms 120, 120 is smaller than the outer diameter dimension of the protection sleeve 121. Therefore, displacement of the protection sleeve 121 to the proximal end side is restrained by the stopper claws 122, 122. Also, the shutter member 140 is abutted against the protection sleeve 121 from a side. Then, the coil spring 146 is compressed, whereby the coil spring 146 exerts an urging force on the shutter member 140 to displace the shutter member 140 radially inward in the inner needle unit 148. Specifically, in the present embodiment, the urging means for urging the shutter member 140, which is the connection member, to the side of the inner needle 18 is constituted by the coil spring 146.

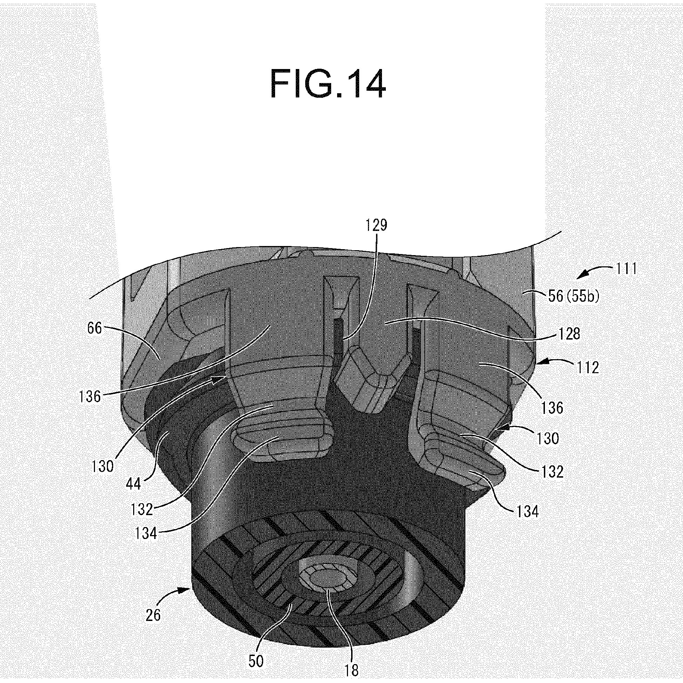

By inserting the inner needle 18 of the inner needle unit 148 through the outer needle unit 14, the outer needle unit 14 and the inner needle unit 148 are assembled to each other, thereby constituting the indwelling needle assembly 110 of this embodiment. Then, the positioning protrusion 128 provided in the cover member 112 is inserted in a cut concavity 129 provided in the lock part 44 of the outer needle hub 26, in contact with the outer peripheral face of the outer needle hub 26, as FIG. 14 shows. This limits relative rotation between the needle tip protector 111 (the inner needle unit 148) and the outer needle unit 14.

The engagement pieces 130, 130 provided on the both circumferential sides of the positioning protrusion 128 extend out to the outside of the outer needle hub 26, and the claws 132, 132 of the engagement pieces 130, 130 are engaged in the lock part 44 provided on the outer peripheral face of the proximal end side opening part 43 of the outer needle hub 26. The distal end of the base part 142 of the shutter member 140 extends out from the needle tip protector 111 to the inside of the outer needle hub 26 while being abutted against the inner peripheral face of the outer needle hub 26. By so doing, the peripheral wall of the outer needle hub 26 is clamped and hold between the positioning protrusion 128 and the base part 142 of the shutter member 140 in the needle tip protector 111. The outer peripheral engagement part of this embodiment is constituted by including these positioning protrusion 128 and engagement pieces 130, 130.

The inside fitting part 80 of the cover member 112 is abutted on the inner peripheral face of the proximal end side opening part 43 of the outer needle hub 26. Specifically, in the present embodiment, the clamping of the peripheral wall of the outer needle hub 26 by these positioning protrusion 128 and base part 142, engagement in the lock part 44 by the claws 132, 132, and contact of the inside fitting part 80 with the inner peripheral face of the outer needle hub 26 constitute the connection mechanism wherein the proximal end side of the outer needle hub 26 is connected to the distal end side of the needle tip protector 111. In the initial state, this connection mechanism controls relative movement for the needle tip protector 111 and the outer needle hub 26.

By drawing the inner needle 18 out of the outer needle unit 14 of the indwelling needle assembly 110 having the above-referred structure, as FIG. 13B shows, the large-diameter parts 29, 29 of the inner needle 18 are engaged in the opening peripheral edge of the passage hole 152 in the inside projection 150 of the protection sleeve 121. With this engaged state, the protection sleeve 121 moves to the proximal end side, accompanying the inner needle 18. After that, as FIG. 13A shows, the inside projection 150 of the protection sleeve 121 is abutted against the inclines 124, 124, which are the distal end side end faces of the stopper claws 122, 122 in the retaining arms 120, 120.

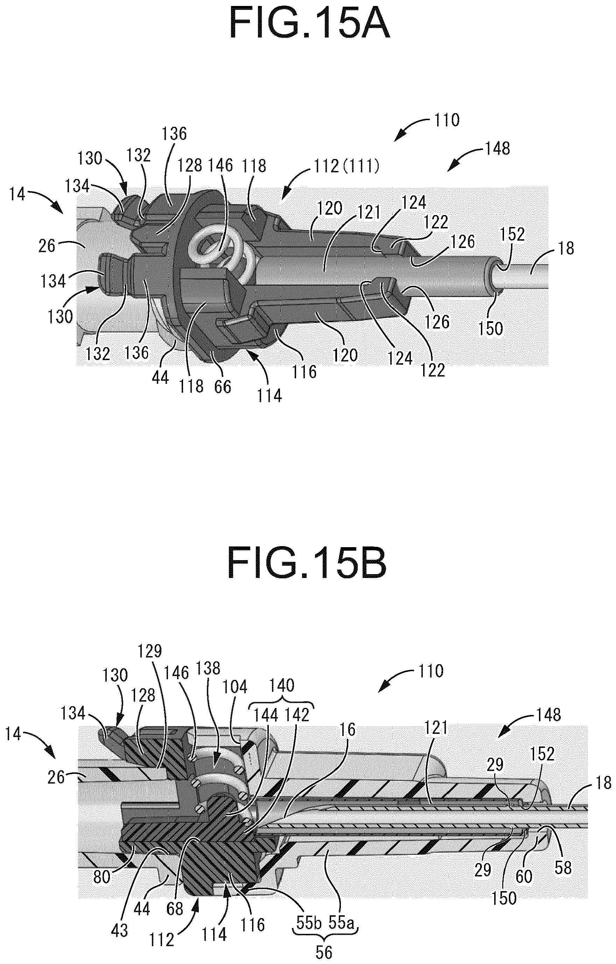

By further drawing the inner needle 18 out after that, as FIGS. 15A and 15B show, the retaining arms 120, 120 are elastically deformed by the guide action of the inclines 124, 124 such that the protruding distal ends expand while using the protruding proximal ends (the connection parts with the guide side walls 118, 118) as fulcrums. This allows the protection sleeve 121 to pass between the stopper claws 122, 122. FIGS. 15A and 15B show the state after operation of the disconnection mechanism, i.e., the same state as FIG. 11 in the above-mentioned first embodiment. FIG. 15A is a perspective view through the protector main body 56, while FIG. 15B is a longitudinal cross sectional view of FIG. 15A, including the protector main body 56.

The inner needle 18 is further extracted to the proximal end side, so that the inside projection 150 of the protection sleeve 121 is abutted against the bottom wall 60 of the protector main body 56, while the contact of the protection sleeve 121 and the shutter member 140 is released. The shutter member 140 is moved to the inner peripheral side, yielding to the urging force of the coil spring 146. By so doing, the distal end side of the inner needle 18 is obstructed by the shutter member 140, thereby protecting the needle tip 16 of the inner needle 18.

In addition to the protection of the needle tip 16, the shutter member 140 moves to the inner peripheral side, releasing the clamping of the peripheral wall of the outer needle hub 26 by the shutter member 140, the positioning protrusion 128, and the engagement pieces 130, 130. Specifically, a gap is formed between the shutter member 140 and the outer needle hub 26, and, using this gap, it is possible to displace the inner needle unit 148 relative to the outer needle unit 14 in the diametrical direction. Therefore, the engagement of the claws 132, 132 in the lock part 44 can be released, whereby the connection between the needle tip protector 111 and the outer needle hub 26 can be released. Particularly, the engagement pieces 130, 130 have a nearly constant thickness dimension across about the whole axial length, and readily undergo flexure to the outside, while having inclines in the inner faces, so that, if the inner needle unit 148 is extracted as parallel to the proximal end side relative to the outer needle unit 14, it is possible to easily release the engagement of the claws 132, 132 in the lock part 44 without feeling resistance.

Also in the indwelling needle assembly 110 of this embodiment structured in this way, the connection mechanism between the needle tip protector 111 and the outer needle hub 26 is released at the same time as the protection of the inner needle 18, thereby improving operation easiness and manipulation easiness. Especially, the connection mechanism is released by the displacement of the shutter member 140 to the inner peripheral side, thereby avoiding the enlargement of the indwelling needle assembly 110.

For the needle tip protector 111 of the present embodiment, before the extraction of the inner needle 18, the protection sleeve 121 is avoided from being displaced to the proximal end side further than the stopper claws 122, 122. Specifically, if the protection sleeve moves to the proximal end side and the contact of the connection member and the protection sleeve is released before the extraction of the inner needle, the connection member may be moved to the inner peripheral side by the urging means, which may lead to contact between the connection member and the inner needle. In this case, there is the risk that the extracted inner needle may slide along the connection member, and the extraction resistance of the inner needle may become greater. However, this risk can be avoided by preventing the displacement of the protection sleeve 121 to the proximal end side before the extraction of the inner needle 18 like the present embodiment.

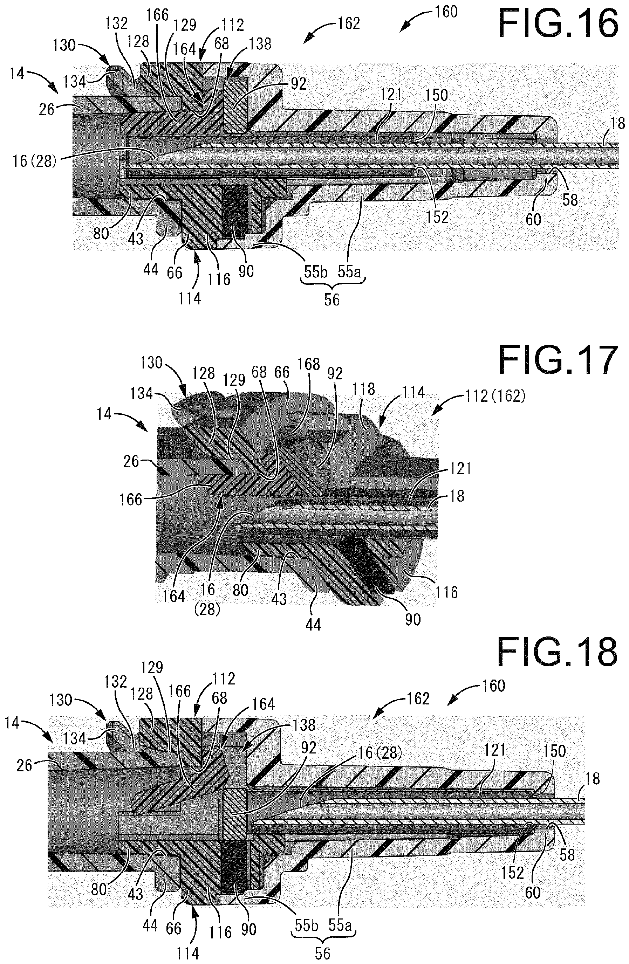

Next, FIGS. 16 and 17 show the principal part of an indwelling needle assembly 160 as a third embodiment of this invention. For a needle tip protector 162 of the present embodiment, the same cover member 112 as that of the aforesaid second embodiment is used. However, different members are housed in the inner space 138 defined by the protector main body 56 and the cover member 112. That is, the fixation member 90 and the shutter member 92 in the first embodiment are stored and disposed in the inner space 138, while a tilting member 164 serving as a connection member that can be tilted to the inner peripheral side by the extraction of the inner needle 18 is provided therein.

Specifically, the tilting member 164 has a shape including a base part 166 of about "L" character shape and a pair of support shafts 168, 168 provided at the base part 166 while projecting to both sides of an axis-perpendicular direction (both sides of the near-distant direction of the paper face in FIG. 16). The tilting member 164 is supported between the cover member 112 and the protector main body 56, and the distal end side thereof is allowed to tilt downward in FIG. 16 around the support shafts 168, 168. In the state before the operation of the disconnection mechanism as shown in FIGS. 16 and 17, the base part 166 extends out from the central hole 68 to the distal end side, while it is clamped and positioned between the peripheral wall of the outer needle hub 26 and the protection sleeve 121. FIGS. 16 and 17 show views of the same state as FIG. 10 for the above-mentioned first embodiment.

In short, in the state before the operation of the disconnection mechanism, since the shutter member 92 is fitted in the space provided between the protector main body 56 and the proximal end side part of the base part 166, the base part 166 is avoided from tilting to the lower side in FIG. 16 (a direction of approaching the inner needle 18). Thus, the peripheral wall of the outer needle hub 26 is clamped by the base part 166, the positioning protrusion 128, and the engagement pieces 130, 130 so that the needle tip protector 162 and the outer needle hub 26 are connected. Also in this embodiment, the outer peripheral engagement part is constituted by the positioning protrusion 128 and the engagement pieces 130, 130.

As FIG. 18 shows, since the contact between the shutter member 92 and the protection sleeve 121 is released by extracting the inner needle 18 to the proximal end side, the shutter member 92 is attracted by the fixation member 90 and moved to on the needle axis of the inner needle 18. Accompanying this, the fixation of the tilting member 164 (the base part 166) owing to fitting entry of the shutter member 92 is released, so that the tilting member 164 is displaced to the side of the inner needle 18 around the support shafts 168, 168. Especially in the tilting member 164, the insertion portion into the outer needle hub 26, which is positioned with a large distance from the support shaft 168, effectively moves to the inner peripheral side (the lower side in FIG. 18) within the outer needle hub 26. This releases the clamping of the outer needle hub 26 by the base part 166, and the positioning protrusion 128 and the engagement pieces 130, 130, thereby making it possible to release the connection between the needle tip protector 162 and the outer needle hub 26.

With this embodiment, the tilting member 164 is provided on the side corresponding to the blade face 28 of the inner needle 18 on the circumference, and the indwelling needle assembly 160 of the present embodiment is normally used orienting the upper side in FIG. 16 to the actual upper side. Therefore, it is also possible that the tilting of the tilting member 164 around the support shafts 168, 168 is made by gravitation. Alternatively, it is possible that the shutter member 92 and the base part 166 are connected so that the tilting member 164 is tilted accompanying the movement of the shutter member 92. The tilting member 164 may be assembled to the cover member 112 with a retaining force of such a level the tilting member 164 is not tilted by gravitation. For example in the case where the needle tip protector 162 is removed from the outer needle hub 26, it is possible that, when the needle tip protector 162 is moved to the upper side in FIG. 18 relative to the outer needle hub 26, the connection between the outer needle hub 26 and the needle tip protector 162 is configured to be released while pushing the tilting member 164 to the inner peripheral side using the peripheral wall of the outer needle hub 26.

In the indwelling needle assembly 160 of this embodiment having the aforementioned structure, the same effect as those of the above-described embodiments can be exhibited.

The embodiments of the present invention have been described above, but the present invention is not limited by the specific description of the embodiments. Other embodiments including various changes, amendments, modifications, and the like on the basis of the knowledge of the skilled person can be used to realize the present invention. As long as these embodiments do not deviate from the concept of this invention, they are all included in the range of the present invention.

For example, in the aforesaid first embodiment, the engagement piece 82 extends out to the outside of the outer needle hub 26 from the needle tip protector 22 so that the claw 84 is engaged in the lock part 44, while the claw 84 and the distal end protruding part 98 of the slide member 94 clamp the peripheral wall of the outer needle hub 26, thereby connecting the needle tip protector 22 and the outer needle hub 26. Also, in the aforementioned second and third embodiments, the peripheral wall of the outer needle hub 26 is clamped from outside and inside by the positioning protrusion 128, the engagement pieces 130, 130, and the connection member, thus connecting the needle tip protector 111, 162 and the outer needle hub 26. However, this invention is not limited to these embodiments. Specifically, this invention is not limited to embodiments wherein the needle tip protector and the outer needle hub 26 are connected on the outside of the outer needle hub 26, but the needle tip protector and the outer needle hub 26 may be connected on the inside of the outer needle hub 26.

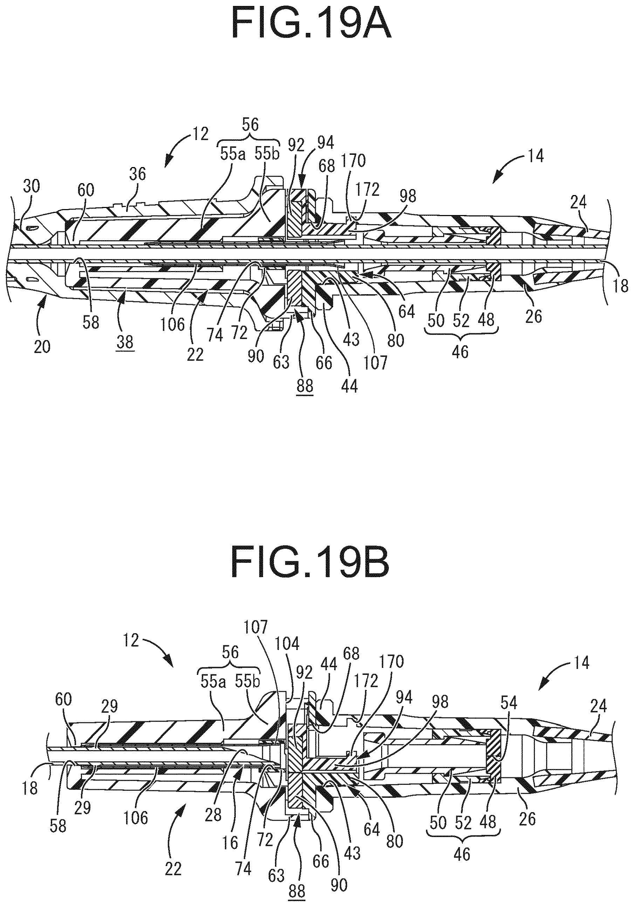

Specifically, as FIG. 19A shows, a mating convex part 170 projecting toward the deeper part of the outer needle hub 26 from the distal end protruding part 98 of the slide member 94 may be provided, while a mating concave part 172 corresponding to the mating convex part 170 may be provided in the inner peripheral face of the outer needle hub 26 so that concave and convex mating of them can connect the needle tip protector 22 and the outer needle hub 26. Then, the inside fitting part 80 positioned on the opposite side to the distal end protruding part 98 in the diametrical direction is positioned to be pushed against the inner peripheral face of the outer needle hub 26. As a result, a restricting force of the relative movement is exerted on the inner peripheral face of the outer needle hub 26, whereby the needle tip protector 22 and the outer needle hub 26 can be connected. Specifically, in this embodiment, the concave and convex mating of the mating convex part 170 and the mating concave part 172 constitutes the connection mechanism connecting the needle tip protector 22 and the outer needle hub 26.

Alternatively, it is possible to provide the inside fitting part 80 with a mating convex part that projects to the outer peripheral side while providing the inner peripheral face of the outer needle hub 26 with a mating concave part that corresponds to the mating convex part. In this case, the distal end protruding part 98 of the slide member 94 located on the opposite side to the inside fitting part 80 in the diametrical direction is pushed against the inner peripheral face of the outer needle hub 26, whereby the needle tip protector 22 and the outer needle hub 26 can be connected, by their relative movement being restricted from the inside of the outer needle hub 26. Also, it is possible as well to provide both the inside fitting part 80 and the distal end protruding part 98 of the slide member 94 with mating convex parts that protrude to the outer peripheral side, while providing the inner peripheral face of the outer needle hub 26 with mating concave parts that correspond to the mating convex parts.

For example, with the aforesaid embodiment shown in FIG. 19A, by extracting the inner needle 18 from the outer needle 24 to the proximal end side, the slide member 94 is made movable to the inner peripheral side, as shown in FIG. 19B. Also in the present embodiment, the slide member 94 moves to the inner peripheral side in this way, thereby forming a gap that enables relative movement of the inner needle unit 12 and the outer needle unit 14 in the diametrical direction, whereby it is possible to release the concave and convex mating of the mating convex part 170 and the mating concave part 172 in the inside of the outer needle hub 26, namely the connection between the needle tip protector 22 and the outer needle hub 26. As a result, the inner needle unit 12 can be removed from the outer needle unit 14. The same goes for the case where the mating convex part is provided for the inside fitting part 80, and the case where it is provided for both the distal end protruding part 98 and the inside fitting part 80.

Also in the case the engagement piece 82, 130 projecting to the outside of the outer needle hub 26 is provided like the above-described embodiments, the connection between the needle tip protector 22, 111, 162 and the outer needle hub 26 in the outside of the outer needle hub 26 is not limited to the engagement between the claw 84, 132 and the lock part 44. Specifically, the lock part is not indispensable for the outer peripheral face of the outer needle hub, and it is possible to provide a concave part corresponding to the claw 84, 132 on the outer peripheral face of the outer needle hub so that the claw 84, 132 of the engagement piece 82, 130 enters in the concave part to be engaged therein. Alternatively, instead of providing the claw, it is possible to use a connection mechanism that keeps the connected state between the outer needle hub and the needle tip protector by restricting the movement of the outer needle hub relative to the needle tip protector, on the basis of a friction force between the engagement piece and the outer peripheral face of the outer needle hub, and a friction force between the distal end protruding part 98 of the slide member 94 in the first embodiment or the base part 142, 166 in the second and third embodiments and the inner peripheral face of the outer needle hub.

For the first and third embodiments, in the needle tip protector 22, 162, the shutter member 92 is not indispensable. Specifically, under protection by the needle tip protector, the needle tip of the inner needle need not be perfectly covered in the axial direction. For example, by separately providing a movement restriction mechanism that prevents the movement of the inner needle to the distal end side when the needle tip of the inner needle is located in the protector main body, it is possible to avoid the protrusion of the inner needle from the needle tip protector so as to prevent inadvertent pricking. Moreover, in the above-referred first embodiment, the shutter member 92 and the slide member 94 are formed by separate members, but they may be formed integrally, that is, a member serving as the shutter and the slide member whose entirety has a cross section of about "L" character shape may be configured to move to the inner peripheral side, thus covering the needle tip of the inner needle in the axial direction. In this case, like the aforementioned third embodiment, the member serving as both the shutter and the slide member can be formed from permanent magnet, or ferromagnetic material attracted by a magnet. The slide member is not required to move up to such a position that the slide member entirely covers the needle tip of the inner needle in the axial direction, as long as the movement forms a gap that enables disconnection of the needle tip protector and the outer needle hub and hence removal of the inner needle unit from the outer needle unit.

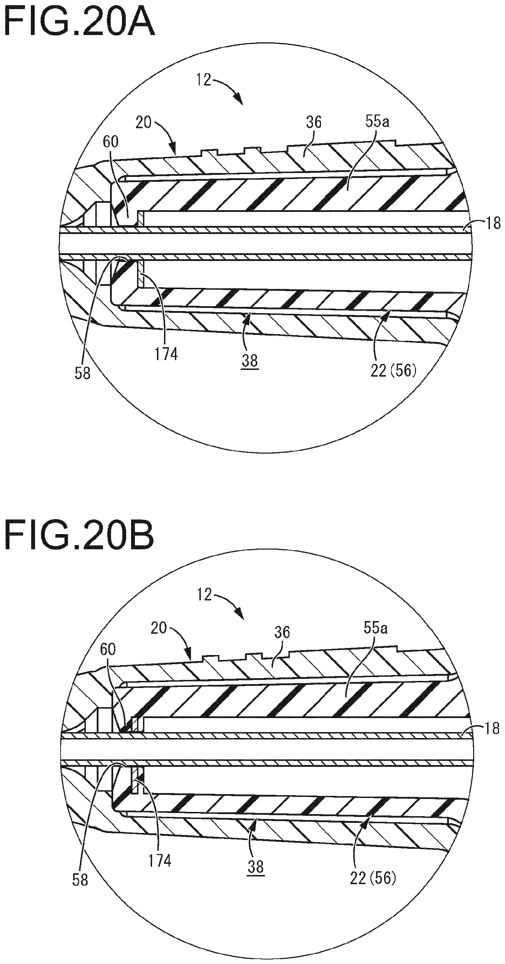

Furthermore, in the present invention, the protection sleeve 106 disposed externally about the inner needle 18 is not always necessary. Specifically, in the above-described embodiment, in the initial state, the movement of the slide member 94 to the inner peripheral side is restricted by contact of the slide member 94 with the protection sleeve 106, but it can be restricted by contact of the slide member with the inner needle. Also, the movement of the inner needle to the proximal end side may be restricted by contact between the proximal end part of the protector main body 56 and the large-diameter parts 29, 29 of the inner needle 18. As FIG. 20A shows, it is possible to fix a washer 174 at the proximal end part of the protector main body 56 so that the washer 174 is abutted on the large-diameter parts 29, 29 of the inner needle 18. It is also possible to fix the washer 174 by insert molding at the proximal end part of the protector main body 56, as FIG. 20B shows. Use of this washer 174 can resolve the problem about dimensional stability of the protector main body 56 where the protector main body 56 is a resin member.

In the above-mentioned first embodiment, the urging means, which urges the slide member 94 to the inner peripheral side of the outer needle hub 26, is constituted by including the shutter member 92 formed of permanent magnet, but the urging means is not limited to this embodiment. That is, as a member constituting the urging means, it is possible to adopt a member that exerts the elastic force as the urging force like the second embodiment such as a metal spring, a resin spring, and a rubber elastic body, as well as the magnet exerting the magnetic force as the urging force like the first embodiment, so as to assemble it in the needle tip protector 22 with the urging force exerted on the slide member 94. The shape of the shutter member 92 is not particularly limited. However, a ball shape is not preferable because the needle tip is likely to be exposed when the ball-shaped shutter member is used, and the shutter member 92 is preferred to have a rectangular cross section, for example.

For the first embodiment, the urging means, which urges the slide member 94 to the inner peripheral side of the outer needle hub 26, is provided so that release of the restriction against the movement of the slide member 94 to the inner peripheral side enables the slide member 94 to automatically move to the inner peripheral side of the outer needle hub 26. However, it is also possible to move the slide member to the inner peripheral side by hand manipulation, instead of employing this urging means. Specifically, it is possible for example that the slide member (the connection member) is assembled axially between the protector main body and the cover member with some clamping force, in short, such that it is not freely movable to the inner peripheral side of the outer needle hub, while the user pushes the slide member inward via the opening window provided at the outer peripheral face of the needle tip protector, thereby moving the slide member to the inner peripheral side. This makes it possible to disconnect the needle tip protector and the outer needle hub at timing at the user's disposal after the extraction of the inner needle from the outer needle unit.

Furthermore, the hemostasis valve mechanism is not limited to the ones disclosed in the aforesaid embodiments. For example, the pusher 50 and the pusher guide 52 are not indispensable.

For the indwelling needle assembly 10, 110, 160 described in the above-mentioned embodiments, it is preferable that the inner needle hub 20 and the needle tip protector 22, 111, 162 cannot be rotated relative to each other. Owing to this, for example during use of the indwelling needle assembly, it is possible to position the connection member and the check window in a prescribed position on the circumference such as the opposite side to the skin of the patient (the upper side in FIG. 4), thereby making it possible to visually check the movement of the connection member easily. By positioning the connection member and the check window on about the opposite side to the patient's skin so that the connection member can be moved to generally the side of the patient's skin, these connections are made to be released by sliding the needle tip protector relative to the outer needle hub to the direction of separation from the patient's skin after the user moves the inner needle to the proximal end side. Therefore, the user can more easily perform the manipulation for releasing these connections. The inner needle hub and the needle tip protector may be made relatively non-rotatable for example by providing a convex part and a concave part for different faces out of the inner peripheral face of the cover tubular part in the inner needle hub and the outer peripheral face of the small-diameter tubular part in the needle tip protector, so as to use concave and convex mating of these concave and convex parts. Alternatively, the inner needle hub and the needle tip protector can be made relatively non-rotatable by the cover tubular part and the small-diameter tubular part having rectangular cross sections corresponding to each other.

### KEYS TO SYMBOLS

10, 110, 160: Indwelling needle assembly; 16: Needle tip; 18: Inner needle; 20: Inner needle hub; 22, 111, 162: Needle tip protector; 24: Outer needle; 26: Outer needle hub; 28: Blade face; 74: Distal end side needle insertion hole (insertion hole); 82, 130: Engagement piece; 84, 132: Claw; 92: Shutter member; 94: Slide member (connection member); 104: Opening window (check window); 106, 121: Protection sleeve; 140: Shutter member (connection member); 164: Tilting member (connection member); 170: Mating convex part; 172: Mating concave part

## Claims

1. An indwelling needle assembly (10, 110, 160) comprising:
an inner needle (18) having an inner needle hub (20) on a proximal end side thereof;
an outer needle (24) having an outer needle hub (26) on a proximal end side thereof, the outer needle (24) receiving the inner needle (18) being inserted from a proximal end side of the outer needle hub (26);
a needle tip protector (22, 111, 162) attached to the inner needle (18), the needle tip protector (22, 111, 162) configured to move to a distal end of the inner needle (18) so as to cover a needle tip (16) of the inner needle (18); and
a connection mechanism connecting the proximal end side of the outer needle hub (26) to a distal end side of the needle tip protector (22, 111, 162),:
a connection member (94, 140, 164) is provided extending out from the needle tip protector (22, 111, 162) to an inside of the outer needle hub (26) while being abutted against an inner face of the outer needle hub (26) so as to restrict movement of the outer needle hub (26) relative to the needle tip protector (22, 111, 162) and to keep a connected state of the outer needle hub (26) to the needle tip protector (22, 111, 162) by the connection mechanism,
a disconnection mechanism is provided and configured to move the connection member (94, 140, 164) to an inner peripheral side of the outer needle hub (26) so as to release the connected state when the inner needle (18) is extracted from the outer needle (24), **characterized by**
a proximal end side of the connection member (94, 140, 164) is housed between opposite faces of a pair of guide walls (70,118) provided at the needle tip protector (22,111,162), the connection member (94, 140, 164) being assembled as movable in a diametrical direction relative to the needle tip protector (22,111,162) while being guided by the pair of guide walls (70,118) so that the connection member (94, 140, 164) is configured to be moved to the inner peripheral side of the outer needle hub (26).

2. The indwelling needle assembly (10, 110, 160) according to claim 1, further comprising an urging means (92, 146) provided to always exert an urging force of a direction to move the connection member (94, 140, 164) to the inner peripheral side of the outer needle hub (26).

3. The indwelling needle assembly (10,110,160) according to claim 2, wherein the urging means (92,146) is stored inside the needle tip protector (22,111, 162).

4. The indwelling needle assembly (10, 110, 160) according to any one of claims 1-3, further comprising an outer peripheral engagement part (82, 84, 128, 130) extending out from the needle tip protector (22, 111, 162) to an outside of the outer needle hub (26) so that the outer peripheral engagement part (82, 84, 128, 130) is abutted against an outer face of the outer needle hub (26) as a peripheral wall of the outer needle hub (26) is clamped between the outer peripheral engagement part (82, 84, 128, 130) and the connection member (94, 140, 164).

5. The indwelling needle assembly (10, 110, 160) according to any one of claims 1-4, wherein the needle tip protector (22, 111, 162) has an insertion hole (74) receiving the inner needle (18) being inserted through the insertion hole (74), and a shutter member (92, 140) configured to cover the insertion hole (74) by movement of the needle tip protector (22, 111, 162) to a distal end side of the inner needle (18).

6. The indwelling needle assembly (10, 110) according to claim 5, wherein the shutter member (92, 140) is provided integrally with the connection member (94, 140).

7. The indwelling needle assembly (10, 110, 160) according to any one of claims 1-6, wherein
a protection sleeve (106, 121) receiving the inner needle (18) being inserted through the protection sleeve (106, 121) is assembled to the needle tip protector (22, 111, 162) in a movable manner in an axial direction, and movement of the connection member (94, 140, 164) to the inner peripheral side of the outer needle hub (26) is restricted by contact of the connection member (94, 140, 164) with an outer peripheral face of the protection sleeve so as to keep the connected state of the outer needle hub to the needle tip protector by the connection member, and
the disconnection mechanism includes an interlocking mechanism interlocking the protection sleeve (106, 121) with the inner needle (18) being extracted from the outer needle (24) to move the protection sleeve within the needle tip protector to a proximal end side of the needle tip protector and releasing restriction against the movement of the connection member (94, 140, 164) by the contact of the connection member with the protection sleeve (106, 121) so that the connection member is moved to the inner peripheral side so as to release the connected state.

8. The indwelling needle assembly (10, 110, 160) according to any one of claims 1-7, wherein the needle tip protector (22, 111, 162) has a check window (104) permitting a visual check of a moved position of the connection member (94, 140, 164).

9. The indwelling needle assembly (10, 110, 160) according to any one of claims 1-8, wherein movement of the connection member (94, 140, 164) to the inner peripheral side of the outer needle hub (26) is realized by the connection member (94, 140, 164) moving to one side in a diametrical direction of the outer needle hub (26).

## Patentansprüche

1. Dauernadelanordnung (10, 110, 160), umfassend:
eine Innennadel (18), die einen Innennadelansatz (20) an einer proximalen Endseite davon aufweist;
eine Außennadel (24), die einen Außennadelansatz (26) an einer proximalen Endseite davon aufweist, wobei die Außennadel (24) die Innennadel (18), die von einer proximalen Endseite des Außennadelansatzes (26) eingesetzt wird, aufnimmt;
einen Nadelspitzenschutz (22, 111, 162), der an der Innennadel (18) befestigt ist, wobei der Nadelspitzenschutz (22, 111, 162) so konfiguriert ist, dass er sich zu einem distalen Ende der Innennadel (18) bewegt, um eine Nadelspitze (16) der Innennadel (18) abzudecken; und
einen Verbindungsmechanismus, der die proximale Endseite des Außennadelansatzes (26) mit einer distalen Endseite des Nadelspitzenschutzes (22, 111, 162) verbindet;
wobei ein Verbindungselement (94, 140, 164) vorgesehen ist, das sich vom Nadelspitzenschutz (22, 111, 162) zu einer Innenseite des Außennadelansatzes (26) erstreckt, während es an einer Innenfläche des Außennadelansatzes (26) anliegt, um Bewegung des Außennadelansatzes (26) relativ zu dem Nadelspitzenschutz (22, 111, 162) einzuschränken und einen verbundenen Zustand des Außennadelansatzes (26) mit dem Nadelspitzenschutz (22, 111, 162) durch den Verbindungsmechanismus aufrechtzuerhalten,
ein Trennmechanismus vorgesehen und so konfiguriert ist, dass er das Verbindungselement (94, 140, 164) zu einer Innenumfangsseite des Außennadelansatzes (26) bewegt, um den verbundenen Zustand zu lösen, wenn die Innennadel (18) aus der Außennadel (24) herausgezogen wird, **dadurch gekennzeichnet, dass**
eine proximale Endseite des Verbindungselements (94, 140, 164) zwischen gegenüberliegenden Flächen eines Paares von Führungswänden (70, 118) untergebracht ist, die am Nadelspitzenschutz (22, 111, 162) vorgesehen sind, wobei das Verbindungselement (94, 140, 164) so montiert ist, dass es in einer diametralen Richtung relativ zum Nadelspitzenschutz (22, 111, 162) beweglich ist, während es durch das Paar von Führungswänden (70, 118) geführt wird, sodass das Verbindungselement (94, 140, 164) so konfiguriert ist, dass es zur Innenumfangsseite des Außennadelansatzes (26) bewegt wird.

2. Dauernadelanordnung (10, 110, 160) nach Anspruch 1, die weiter ein Vorspannmittel (92, 146) umfasst, das dazu vorgesehen ist, stets eine Vorspannkraft in einer Richtung auszuüben, um das Verbindungselement (94, 140, 164) zu der Innenumfangsseite des Außennadelansatzes (26) zu bewegen.

3. Dauernadelanordnung (10, 110, 160) nach Anspruch 2, wobei das Vorspannmittel (92, 146) innerhalb des Nadelspitzenschutzes (22, 111, 162) untergebracht ist.

4. Dauernadelanordnung (10, 110, 160) nach einem der Ansprüche 1-3, die weiter einen Außenumfangseingriffsteil (82, 84, 128, 130) umfasst, der sich aus dem Nadelspitzenschutz (22, 111, 162) zu einer Außenseite des Außennadelansatzes (26) erstreckt, sodass der Außenumfangseingriffsteil (82, 84, 128, 130) an einer Außenfläche des Außennadelansatzes (26) anliegt, wenn eine Umfangswand des Außennadelansatzes (26) zwischen dem Außenumfangseingriffsteil (82, 84, 128, 130) und dem Verbindungselement (94, 140, 164) eingeklemmt wird.

5. Dauernadelanordnung (10, 110, 160) nach einem der Ansprüche 1-4, wobei der Nadelspitzenschutz (22, 111, 162) ein Einsatzloch (74), das die Innennadel (18) aufnimmt, die durch das Einsatzloch (74) eingesetzt wird, und ein Verschlusselement (92, 140) aufweist, das so konfiguriert ist, dass es das Einsatzloch (74) durch Bewegung des Nadelspitzenschutzes (22, 111, 162) zu einer distalen Endseite der Innennadel (18) abdeckt.

6. Dauernadelanordnung (10, 110) nach Anspruch 5, wobei das Verschlusselement (92, 140) einstückig mit dem Verbindungselement (94, 140) vorgesehen ist.

7. Dauernadelanordnung (10, 110, 160) nach einem der Ansprüche 1-6, wobei
eine Schutzhülse (106, 121), die die Innennadel (18) aufnimmt, die durch die Schutzhülse (106, 121) eingesetzt wird, auf eine bewegliche Weise in einer axialen Richtung am Nadelspitzenschutz (22, 111, 162) montiert ist, und Bewegung des Verbindungselements (94, 140, 164) zur Innenumfangsseite des Außennadelansatzes (26) durch Kontakt des Verbindungselements (94, 140, 164) mit einer Außenumfangsfläche der Schutzhülse begrenzt wird, um den verbundenen Zustand des Außennadelansatzes mit dem Nadelspitzenschutz durch das Verbindungselement aufrechtzuerhalten, und
der Trennmechanismus einen Verriegelungsmechanismus einschließt, der die Schutzhülse (106, 121) mit der Innennadel (18) verriegelt, die aus der Außennadel (24) herausgezogen wird, um die Schutzhülse innerhalb des Nadelspitzenschutzes zu einer proximalen Endseite des Nadelspitzenschutzes zu bewegen, und eine Einschränkung der Bewegung des Verbindungselements (94, 140, 164) durch den Kontakt des Verbindungselements mit der Schutzhülse (106, 121) löst, sodass das Verbindungselement zur Innenumfangsseite bewegt wird, um den verbundenen Zustand zu lösen.

8. Dauernadelanordnung (10, 110, 160) nach einem der Ansprüche 1-7, wobei der Nadelspitzenschutz (22, 111, 162) ein Kontrollfenster (104) aufweist, das eine visuelle Überprüfung einer bewegten Position des Verbindungselements (94, 140, 164) ermöglicht.

9. Dauernadelanordnung (10, 110, 160) nach einem der Ansprüche 1-8, wobei Bewegung des Verbindungselements (94, 140, 164) zur Innenumfangsseite des Außennadelansatzes (26) realisiert wird, indem sich das Verbindungselement (94, 140, 164) in einer diametralen Richtung des Außennadelansatzes (26) zu einer Seite bewegt.

## Revendications

1. Ensemble aiguille à demeure (10, 110, 160) comprenant :
une aiguille interne (18) présentant un raccord d'aiguille interne (20) sur un côté extrémité proximale de celle-ci ;
une aiguille externe (24) présentant un raccord d'aiguille externe (26) sur un côté extrémité proximale de celle-ci, l'aiguille externe (24) recevant l'aiguille interne (18) qui est insérée à partir d'un côté extrémité proximale du raccord d'aiguille externe (26) ;
un dispositif de protection de pointe d'aiguille (22, 111, 162) fixé à l'aiguille interne (18), le dispositif de protection de pointe d'aiguille (22, 111, 162) étant configuré pour se déplacer vers une extrémité distale de l'aiguille interne (18) de manière à recouvrir une pointe d'aiguille (16) de l'aiguille interne (18) ; et
un mécanisme de liaison reliant le côté extrémité proximale du raccord d'aiguille externe (26) à un côté extrémité distale du dispositif de protection de pointe d'aiguille (22, 111, 162),
un élément de liaison (94, 140, 164) est prévu s'étendant depuis le dispositif de protection de pointe d'aiguille (22, 111, 162) jusqu'à un interne du raccord d'aiguille externe (26) tout en étant en butée contre une face interne du raccord d'aiguille externe (26) de manière à restreindre un mouvement du raccord d'aiguille externe (26) par rapport au dispositif de protection de pointe d'aiguille (22, 111, 162) et à maintenir un état relié du raccord d'aiguille externe (26) au dispositif de protection de pointe d'aiguille (22, 111, 162) par le mécanisme de liaison,
un mécanisme de débranchement est prévu et configuré pour déplacer l'élément de liaison (94, 140, 164) vers un côté périphérique interne du raccord d'aiguille externe (26) de sorte à libérer l'état relié lorsque l'aiguille interne (18) est extraite de l'aiguille externe (24), **caractérisé en ce que**
un côté extrémité proximale de l'élément de liaison (94, 140, 164) est logé entre des faces opposées d'une paire de parois de guidage (70, 118) disposées au niveau du dispositif de protection de pointe d'aiguille (22, 111, 162), l'élément de liaison (94, 140, 164) étant assemblé de manière mobile dans une direction diamétrale par rapport au dispositif de protection de pointe d'aiguille (22, 111, 162) tout en étant guidé par la paire de parois de guidage (70, 118) de telle sorte que l'élément de liaison (94, 140, 164) soit configuré pour être déplacé vers le côté périphérique interne du raccord d'aiguille externe (26).

2. Ensemble aiguille à demeure (10, 110, 160) selon la revendication 1, comprenant en outre un moyen de poussée (92, 146) disposé à toujours exercer une force de poussée dans une direction de déplacement de l'élément de liaison (94, 140, 164) vers le côté périphérique interne du raccord d'aiguille externe (26).

3. Ensemble aiguille à demeure (10, 110, 160) selon la revendication 2, dans lequel le moyen de poussée (92, 146) est stocké à l'intérieur du dispositif de protection de pointe d'aiguille (22, 111, 162).

4. Ensemble aiguille à demeure (10, 110, 160) selon l'une quelconque des revendications 1-3, comprenant en outre une partie de mise en prise périphérique externe (82, 84, 128, 130) s'étendant à partir du dispositif de protection de pointe d'aiguille (22, 111, 162) vers un extérieur du raccord d'aiguille externe (26) de telle sorte que la partie de mise en prise périphérique externe (82, 84, 128, 130) vienne en butée contre une face externe du raccord d'aiguille externe (26) au fur et à mesure qu'une paroi périphérique du raccord d'aiguille externe (26) est serrée entre la partie de mise en prise périphérique externe (82, 84, 128, 130) et l'élément de liaison (94, 140, 164).

5. Ensemble aiguille à demeure (10, 110, 160) selon l'une quelconque des revendications 1-4, dans lequel le dispositif de protection de pointe d'aiguille (22, 111, 162) présente un trou d'insertion (74) recevant l'aiguille interne (18) qui est insérée à travers le trou d'insertion (74), et un élément d'obturation (92, 140) configuré pour recouvrir le trou d'insertion (74) par un mouvement du dispositif de protection de pointe d'aiguille (22, 111, 162) vers un côté extrémité distale de l'aiguille interne (18).

6. Ensemble aiguille à demeure (10, 110) selon la revendication 5, dans lequel l'élément d'obturation (92, 140) est disposé d'un seul tenant avec l'élément de liaison (94, 140).

7. Ensemble aiguille à demeure (10, 110, 160) selon l'une quelconque des revendications 1-6, dans lequel
un manchon de protection (106, 121) recevant l'aiguille interne (18) insérée à travers le manchon de protection (106, 121) est assemblé au dispositif de protection de pointe d'aiguille (22, 111, 162) de manière mobile dans une direction axiale et un mouvement de l'élément de liaison (94, 140, 164) vers le côté périphérique interne du raccord d'aiguille externe (26) est limité par le contact de l'élément de liaison (94, 140, 164) avec une face périphérique externe du manchon de protection de manière à maintenir l'état relié du raccord d'aiguille externe au dispositif de protection de pointe d'aiguille par l'élément de liaison, et
le mécanisme de débranchement inclut un mécanisme de verrouillage verrouillant le manchon de protection (106, 121) avec l'aiguille interne (18) qui est extraite de l'aiguille externe (24) pour déplacer le manchon de protection à l'intérieur du dispositif de protection de pointe d'aiguille vers un côté extrémité proximale du dispositif de protection de pointe d'aiguille et libérant la restriction contre le mouvement de l'élément de liaison (94, 140, 164) par le contact de l'élément de liaison avec le manchon de protection (106, 121) de telle sorte que l'élément de liaison soit déplacé vers le côté périphérique interne de manière à libérer l'état relié.

8. Ensemble aiguille à demeure (10, 110, 160) selon l'une quelconque des revendications 1-7, dans lequel le dispositif de protection de pointe d'aiguille (22, 111, 162) présente une fenêtre de contrôle (104) permettant un contrôle visuel d'une position déplacée de l'élément de liaison (94, 140, 164).

9. Ensemble aiguille à demeure (10, 110, 160) selon l'une quelconque des revendications 1-8, dans lequel un mouvement de l'élément de liaison (94, 140, 164) vers le côté périphérique interne du raccord d'aiguille externe (26) est réalisé par l'élément de liaison (94, 140, 164) se déplaçant vers un côté dans une direction diamétrale du raccord d'aiguille externe (26).
